(19) Europäisches Patentamt
European Patent Office
Office européen des brevets

(11) **EP 3 107 444 B1**

(12) **FASCICULE DE BREVET EUROPEEN**

(45) Date de publication et mention de la délivrance du brevet:
**02.09.2020  Bulletin 2020/36**

(21) Numéro de dépôt: **15704805.9**

(22) Date de dépôt: **17.02.2015**

(51) Int Cl.:
*A61B 5/024* (2006.01)   *A61B 5/0456* (2006.01)
*A61B 5/16* (2006.01)   *A61B 5/0482* (2006.01)
*A61B 5/00* (2006.01)

(86) Numéro de dépôt international:
**PCT/EP2015/053345**

(87) Numéro de publication internationale:
**WO 2015/121503 (20.08.2015 Gazette 2015/33)**

(54) **PROCÉDÉ ET SYSTÈME DE SURVEILLANCE DU SYSTÈME NERVEUX AUTONOME D'UN SUJET**

VERFAHREN UND SYSTEM ZUR ÜBERWACHUNG DES AUTONOMEN NERVENSYSTEMS EINER PERSON

METHOD AND SYSTEM FOR MONITORING THE AUTONOMIC NERVOUS SYSTEM OF A SUBJECT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **17.02.2014  FR 1451257**

(43) Date de publication de la demande:
**28.12.2016  Bulletin 2016/52**

(73) Titulaire: **Société Codesna**
**06200 Nice (FR)**

(72) Inventeur: **ZOICAS, Vasile**
**06610 La Gaude (FR)**

(74) Mandataire: **Hautier, Nicolas**
**Cabinet Hautier**
**20, rue de la Liberté**
**06000 Nice (FR)**

(56) Documents cités:
**US-A1- 2007 299 354     US-A1- 2012 136 226**

- **Camillo Cammarota ET AL: "Analysis of Stationary Periods of Heart Rate via Symbolic Dynamics" In: "Field Programmable Logic and Application", 1 janvier 2002 (2002-01-01), Springer Berlin Heidelberg, Berlin, Heidelberg, XP055150278, ISSN: 0302-9743 ISBN: 978-3-54-045234-8 vol. 2526, pages 13-19, DOI: 10.1007/3-540-36104-9_2, pages 13-14 page 16 page 17, ligne 28-30**
- **G. GRAFF ET AL: "Ordinal pattern statistics for the assessment of heart rate variability", THE EUROPEAN PHYSICAL JOURNAL SPECIAL TOPICS, vol. 222, no. 2, 1 juin 2013 (2013-06-01), pages 525-534, XP055150168, ISSN: 1951-6355, DOI: 10.1140/epjst/e2013-01857-4**
- **A. PORTA ET AL: "Temporal asymmetries of short-term heart period variability are linked to autonomic regulation", AMERICAN JOURNAL OF PHYSIOLOGY. REGULATORY, INTEGRATIVE AND COMPARATIVE PHYSIOLOGY, vol. 295, no. 2, 11 juin 2008 (2008-06-11) , pages R550-R557, XP055149893, ISSN: 0363-6119, DOI: 10.1152/ajpregu.00129.2008**

**Description**

DOMAINE TECHNIQUE DE L'INVENTION

La présente invention concerne en général un procédé et un système de surveillance d'un système nerveux autonome (SNA). L'invention propose par ailleurs, un procédé et un système d'aide à la régulation de l'activité du SNA. Elle trouve pour avantage particulier les systèmes de surveillance ou de régulation qui nécessitent un suivi du SNA en temps réel ou tout au moins avec une très bonne réactivité.

ÉTAT DE LA TECHNIQUE

Le système nerveux autonome (SNA) est la partie du système nerveux responsable des fonctions automatiques, non soumises au contrôle volontaire d'un être vivant tel qu'une personne. Le SNA, est composé de deux branches, respectivement le système sympathique et le système parasympathique. Les rôles des systèmes sympathique et parasympathique sont différents et ils exercent généralement des effets antagonistes sur les mêmes organes cibles. D'une manière générale, le système sympathique aide le corps à répondre à des situations d'urgence par accélérer les réactions des neurones afin d'augmenter par exemple la fréquence des battements cardiaques et de ralentir le processus de digestion.

En revanche, le système parasympathique aide le corps à préserver et à restaurer son énergie. Lorsqu'une personne se détend, par exemple en se reposant dans une chaise, le système parasympathique abaisse sa pression sanguine, ralentit la fréquence des battements cardiaques et accélère le processus de digestion.

Un stress prolongé génère une activité émotionnelle négative qui stimule le système sympathique en créant ainsi un déséquilibre constant qui provoque de nombreuses symptômes. Afin de fournir à des chercheurs et médecins des données précises pour effectuer des diagnostics et décider des traitements adaptés, il existe alors un besoin consistant à surveiller de manière fiable et précise les systèmes sympathique et parasympathique et à en donner une représentation aisément interprétable. Une interprétation du SNA peut permettre d'identifier une pathologie particulière comme par exemple un état dans lequel le système sympathique d'un patient est anormalement actif par rapport au système parasympathique. Ainsi, le médecin peut effectuer des diagnostics et décider des remèdes appropriés.

Il existe de nombreuses méthodes pour surveiller et analyser le SNA.

La variabilité de la fréquence cardiaque (VFC ; acronyme de l'anglais « Heart Rate Variability », abréviation HRV) est en général utilisée pour évaluer les fonctions physiologiques telles que les activités des systèmes sympathique et parasympathique. La VFC peut être générée par exemple en utilisant des données d'une séquence d'intervalles d'inter-battements dans un signal physiologique tel qu'un signal d'électrocardiogramme (ECG). Le plus souvent, un intervalle/décalage entre deux battements cardiaques successifs, dénommé « intervalle RR », est défini comme un intervalle de temps entre deux sommets préférentiellement successifs d'une onde R d'un signal ECG.

Il existe plusieurs méthodes d'analyse et de traitement de signal utilisant la VFC, dont la majorité est basée sur une analyse dans le domaine fréquentiel. Néanmoins, les analyses fréquentielles présentent plusieurs inconvénients notamment en termes de précision et de temps d'exécution.

En effet, ces analyses fréquentielles exigent de mettre en oeuvre des fonctions mathématiques telles que la transformée de Fourier rapide habituellement désignée par l'acronyme FFT (de l'anglais « Fast Fourier Transformation »,) ou/et la méthode de démodulation complexe (CDM, acronyme de l'anglais «Complex Démodulation Method »), décrites dans les documents US7079888 et US20130009779A1. Or ces fonctions mathématiques nécessitent un temps d'enregistrement long et ne peuvent donc pas être réalisées en temps réel.

De plus, il est difficile de séparer correctement dans le domaine fréquentiel les données appartenant respectivement au système sympathique et au système parasympathique. Autrement dit, les deux systèmes sympathique et parasympathique ne peuvent pas facilement être analysés de manière indépendante.

Les analyses fréquentielles sont en outre très sensibles aux bruits d'un signal ECG tels que les faux battements générés lors de la capture du signal par exemple dans le cas où la personne se déplace lors d'une activité sportive ou si le capteur ECG ne fonctionne pas correctement. Les analyses fréquentielles sont sensibles également aux battements atypiques tels que les extrasystoles, arythmies (bigéminées, trigéminées), etc. Ainsi, les données transformées dans le domaine fréquentiel sont très fortement faussées du fait de bruits ou de faux battements, ce qui réduit considérablement la précision de la surveillance du SNA. Les analyses fréquentielles basées sur les méthodes connues sont donc très peu fiables dès lors que le patient présente des symptômes entrainant des battements atypiques, ou/et dans le cas où de faux battements sont générés par exemple par un détecteur peu performant ou fonctionnant dans des conditions d'acquisition défavorables.

D'autres solutions proposent des analyses effectuées dans le domaine temporel. Ces solutions connues prévoient, comme c'est le cas dans le document US 20130079652A1, des calculs tels qu'une mesure sélectionnée parmi un ensemble de mesures comprenant un intervalle RR moyen, un écart-type des intervalles RR, un ratio d'écarts-types suivant de différents axes d'un diagramme de dispersion des intervalles RR, une différence entre ratios des écarts-types, un écart-type d'une moyenne d'intervalles RR dans différents segments de temps etc.

Les calculs d'analyses temporelles comme mentionnés ci-dessus peuvent être réalisés en temps réel. En revanche, ces calculs ne permettent pas plus d'analyser les deux systèmes sympathique et parasympathique de manière indépendante. Par exemple, si le système sympathique d'un patient reste actif plus longtemps qu'une période prédéterminée et empêche le système parasympathique de jouer son rôle de récupération, cela ne peut pas être reflété par le résultat de ces calculs d'analyses temporelles. Ces calculs ne permettent pas d'analyser l'état d'équilibre entre les deux systèmes sympathique et parasympathique du SNA et ainsi ne permettent pas de déterminer avec précision le niveau de stress d'un patient.

Par conséquent, il existe un besoin consistant à proposer une solution permettant de fournir une analyse fiable, simple et rapide pour surveiller les systèmes sympathique et parasympathique de manière indépendante, avec une bonne réactivité et de préférence en temps réel et tout en limitant la sensibilité à des facteurs perturbants tels que des battements atypiques et des bruits comme de faux battements générés lors de capture de signaux ECG.

## RÉSUMÉ DE L'INVENTION

Pour atteindre cet objectif, un aspect de la présente invention concerne un procédé de surveillance d'un système nerveux autonome (SNA) suivant la revendication 1.

Le procédé selon l'invention permet d'analyser temporellement les deux branches sympathique et parasympathique du système SNA, de manière indépendante et en temps réel ou tout au moins avec une réactivité améliorée. Il est ainsi possible de rétroagir en temps réel pour modifier l'activité du SNA selon le but recherché : par exemple une réduction du stress ou une augmentation de l'intensité des émotions.

Par ailleurs, le procédé selon l'invention permet de surveiller les systèmes sympathique et parasympathique de manière indépendante et tout en limitant la sensibilité à des facteurs perturbants tels que des battements atypiques et des bruits comme des faux battements.

Dans le cadre de la présente invention, on désigne par sujet toute personne ou animal dont le battement cardiaque peut être détecté.

Au moins les étapes de génération et de calcul sont mises en œuvre par ordinateur, c'est-à-dire qu'elles sont exécutées par au moins un microprocesseur.

L'invention comprend les caractéristiques TAS, TAP et NS. Optionnellement l'invention comprend des caractéristiques complémentaires. Les caractéristiques sont présentées dans les lignes suivantes: Dans les équations suivantes $a_{k-1}$ = valeur de l'intervalle de temps immédiatement précédent l'intervalle de temps de durée $a_k$, soit $a_{k-1} = t_{k-1} - t_{k-2}$, et k = (X-W+2), (X-W+3)... X.

- le $TAS(t_X)$ représentant le niveau d'activité du système sympathique et dans lequel le $TAS(t_X)$ est calculé en appliquant l'équation suivante :

$$TAS(t_X) = 100 \cdot \frac{\sum_{k=X-W+2}^{X} (t_k - t_{k-1}) \ \text{si} \ (a_k \leq a_{k-1})}{\sum_{k=X-W+2}^{X} (t_k - t_{k-1})}$$

- le $TAP(t_X)$ représentant le niveau d'activité du système parasympathique et dans lequel le $TAP(tX)$ est calculé en appliquant l'équation suivante :

$$TAP(t_X) = 100 \cdot \frac{\sum_{k=X-W+2}^{X} (t_k - t_{k-1}) \ \text{si} \ (a_k \geq a_{k-1})}{\sum_{k=X-W+2}^{X} (t_k - t_{k-1})}$$

- le $TPSP(t_X)$ étant calculé en appliquant l'équation suivante :

$$TPSP(t_X) = 100 \cdot \frac{\sum_{k=X-W+2}^{X} (t_k - t_{k-1}) \ \text{si} \ (a_k < a_{k-1} \ \text{et} \ a_{k-1} > a_{k-2} \ \text{et} \ a_{k+1} > a_k)}{\sum_{k=X-W+2}^{X} (t_k - t_{k-1})}$$

Ainsi, le $TPSP(t_X)$ est calculé de manière à obtenir une troisième somme partielle par accumulation des valeurs $a_k$ dont chacune perturbe la tendance monotone croissante formée par des intervalles RR adjacents à l'intervalle RR correspondant à la valeur $a_k$ et à diviser ladite somme troisième somme partielle par la somme de toutes les W-1 valeurs $a_k$ ;

-   le $TPPS(t_X)$ étant calculé en appliquant l'équation suivante :

$$TPPS(t_X) = 100 \cdot \frac{\sum_{k=X-W+2}^{X} (t_k - t_{k-1}) \ \text{si} \ (a_k > a_{k-1} \ \text{et} \ a_{k-1} < a_{k-2} \ \text{et} \ a_{k+1} < a_k)}{\sum_{k=X-W+2}^{X} (t_k - t_{k-1})}$$

Ainsi, le $TPPS(t_X)$ est calculé de manière à obtenir une troisième somme partielle par accumulation des valeurs $a_k$ dont chacune perturbe la tendance monotone décroissante formée par des intervalles RR adjacents à l'intervalle RR correspondant à la valeur $a_k$ et à diviser ladite somme troisième somme partielle par la somme de toutes les W-1 valeurs $a_k$.

De plus, l'invention concerne deux paramètres $\overline{TPSP}$ et $\overline{TPPS}$ comme ci-dessous :

-   le paramètre $\overline{TPSP}$ représentant le taux approximé de pollution du système sympathique vers le système parasympathique et calculé en appliquant l'équation suivante :

$$\overline{TPSP} = 100 \cdot \frac{\sum_{n=1}^{N-1} 1 \ \text{si} \ (a_n < a_{n-1} \ \text{et} \ a_{n-1} > a_{n-2} \ \text{et} \ a_{n+1} > a_n)}{N-1}$$

-   le paramètre $\overline{TPPS}$ représentant le taux approximé de pollution du système parasympathique vers le système sympathique et calculé en appliquant l'équation suivante :

$$\overline{TPPS} = 100 \cdot \frac{\sum_{n=1}^{N-1} 1 \ \text{si} \ (a_n > a_{n-1} \ \text{et} \ a_{n-1} < a_{n-2} \ \text{et} \ a_{n+1} < a_n)}{N-1}$$

De préférence, l'invention concerne en outre une application comprenant au moins le calcul des paramètres $TAS(t_X)$ et $TAP(t_X)$ et comprenant le calcul d'un paramètre $NS(t_X)$ représentatif d'un niveau de stress du sujet à l'instant $t_X$ et calculé en appliquant l'équation suivante :

$$NS(t_X) = 100 + TAS(t_X) - TAP(t_X).$$

De préférence, l'invention concerne une autre application comprenant le calcul d'un paramètre $NSR(t_X)$ relatif un niveau de stress résiduel du sujet à l'instant $t_X$ et calculé en appliquant l'équation suivante :

$$NSR(t_X) = NS(t_X) \cdot \frac{RC_{Repos}}{RC(t_X)}$$

dans laquelle $RC_{Repos}$ est la fréquence cardiaque au repos, c'est-à-dire le sujet est inactif pendant au moins 20 secondes et de préférence 40 secondes, $RC(t_X)$ est la fréquence cardiaque à l'instant $t_X$.

Selon un mode de réalisation préféré, le procédé comprend :

- la génération d'un stimulus de respiration fourni au sujet pendant une durée T, le stimulus de respiration comprenant une consigne respiratoire pour que le sujet respire de manière symétrique ;
- la durée T étant calculée en fonction d'au moins une donnée fonction du rythme cardiaque (RC) du sujet.

Selon un mode de réalisation, l'au moins un paramètre comprend une valeur de stress chronique SC qui est fonction du paramètre $NS(t_X)$ pendant ladite durée T.

Selon un mode de réalisation, la valeur de stress chronique SC est égale à la valeur moyenne du paramètre $NS(t_X)$ sur ladite durée T du stimulus de respiration.

Selon un mode de réalisation, le procédé comprend le calcul et l'affichage d'une corrélation mathématique entre le rythme cardiaque (RC) du sujet et une fonction qui commande le stimulus de respiration.

Selon un mode de réalisation, ladite donnée fonction du rythme cardiaque (RC) est une donnée représentant le rythme cardiaque (RC) du sujet.

Selon un mode de réalisation, le signal physiologique est un signal d'électrocardiogramme (ECG), ou tout autre signal physiologique qui est fonction du rythme cardiaque

L'invention concerne une étape de fourniture d'une représentation visuelle et/ou auditive dudit au moins un paramètre.

De préférence, ledit au moins un paramètre est calculé en temps réel ou à intervalles réguliers et comprend une étape de fourniture d'une représentation visuelle de l'évolution au cours du temps dudit au moins un paramètre. De manière avantageuse, l'étape de fourniture d'une représentation visuelle comprend l'affichage d'un graphique ou d'une barre évoluant au cours du temps.

Dans une autre application de l'invention, l'étape de fourniture d'une représentation visuelle de l'évolution au cours du temps dudit au moins un paramètre comprend l'affichage d'au moins un avatar et/ou d'au moins un objet animé dont l'animation ou l'évolution visuelle est fonction de l'évolution au cours du temps dudit au moins un paramètre.

Selon un mode de réalisation, l'étape d'acquisition d'au moins un signal physiologique est réalisée par au moins un des dispositifs suivants qui sont configurés pour être portés par le sujet: un capteur d'électrocardiogramme (ECG), une ceinture thoracique, un bracelet ou une montre équipés d'un capteur photopléthysmographique.

Selon un mode de réalisation, la fourniture d'une donnée représentative dudit au moins un paramètre comprend un affichage sur écran de l'un parmi des dispositifs suivants : une montre, un téléphone, un ordinateur portable, une tablette.

Selon un mode de réalisation, les paramètres $TAS(t_X)$, $TAP(t_X)$, $TPSP(t_X)$, $TPPS(t_X)$, $NS(t_X)$ et $NSR(t_X)$ peuvent être utilisés dans des jeux vidéo et des simulateurs afin de montrer la condition actuelle du corps d'un joueur telle que le niveau de stress, le niveau d'énergie, ou de simuler une condition virtuelle d'un personnage dans un jeu vidéo ou par un simulateur.

De plus, l'invention concerne un système de surveillance du système nerveux autonome (SNA) suivant la revendication 12.

Le système comprend un dispositif d'affichage, couplé au module de traitement des données et configuré pour fournir au sujet une donnée représentative dudit au moins un paramètre.

Optionnellement, l'invention comprend au moins l'une quelconque des caractéristiques suivantes prises séparément ou en combinaison :

Selon un mode de réalisation, le module de traitement des données est configuré pour réaliser des étapes suivantes :

- la génération d'un stimulus de respiration fourni au sujet pendant une durée T, le stimulus de respiration comprenant une consigne respiratoire pour que le sujet respire de manière symétrique ;
- la durée T étant calculée en fonction d'au moins une donnée fonction du rythme cardiaque (RC) du sujet.

Selon un mode de réalisation, l'au moins un paramètre que le module de traitement des données est configuré pour calculer est une valeur de stress chronique SC, la valeur de stress chronique SC étant fonction du paramètre NS(tX) pendant ladite durée T.

Selon un mode de réalisation, la valeur de stress chronique SC est égale à la valeur moyenne du paramètre NS(tX) pendant ladite durée T du stimulus de respiration.

Selon un mode de réalisation, le capteur est un des dispositifs suivants qui sont configurés pour être portés par le sujet : un capteur d'électrocardiogramme (ECG), une ceinture thoracique, un dispositif photopléthysmographique installé dans un bracelet ou dans une montre.

Selon un mode de réalisation, le module de traitement des données est mécaniquement solidaire du capteur.

Alternativement, le module de traitement des données est localisé à distance du capteur et couplé au capteur en utilisant un module de communication filaire ou sans fil.

Selon un mode de réalisation, le module de traitement des données équipe un appareil électronique tel qu'un téléphone portable, une tablette, un ordinateur, etc.

De préférence, le système de surveillance comprend un détecteur configuré pour acquérir le signal physiologique et un dispositif d'affichage configuré pour afficher une représentation visuelle dudit au moins un paramètre.

De manière encore plus avantageuse, le système de surveillance est configuré de manière à fournir en temps réel et audit sujet une représentation visuelle dudit au moins un paramètre.

L'invention concerne un appareil d'aide à la régulation du SNA d'un sujet comprenant le système de surveillance comme ci-dessus.

L'invention concerne en outre un simulateur comprenant le système de surveillance comme ci-dessus.

BRÈVE DESCRIPTION DES FIGURES

Les buts, objets, ainsi que les caractéristiques et avantages de l'invention ressortiront mieux de la description détaillée d'un mode de réalisation de cette dernière qui est illustré par les dessins d'accompagnement suivants dans lesquels :

La FIGURE 1 illustre un système de surveillance d'un système nerveux autonome (SNA) d'un être vivant tel qu'une personne selon un exemple de mode de réalisation de l'invention.

La FIGURES 2(a) montre un signal ECG comprenant N sommets signifiant respectivement un battement cardiaque de la personne 10 mesuré à un instant $t_0$, $t_1$ à $t_{N-1}$ par le capteur ECG 110 selon un mode de réalisation de l'invention.

La FIGURE 2(b) montre la variabilité de la fréquence cardiaque VFC illustrée par N-1 intervalles RR $a_1$, $a_2$ à $a_{N-1}$ correspondant respectivement aux instants $t_1$, $t_2$ à $t_{N-1}$.

La FIGURE 3 montre des résultats des calculs de deux paramètres TAS et TAP sur une durée d'échantillonnage TE de 100 battements cardiaques (N = 100).

La FIGURE 4 montre les paramètres TAS($t_X$) et TAP($t_X$) obtenus en temps réel par calcul basé sur 30 échantillons de la fenêtre glissante à chaque instant $t_X$ (W = 30). Les FIGURES 5(a) et 5(b) montrent chacune un spectre de la VFC selon deux types de respirations.

La FIGURE 6(a) montre une partie d'un premier signal ECG et sa VFC, le premier signal ECG comprenant 300 battements sans ajout de faux battements cardiaques.

La FIGURE 6(b) montre une partie d'un deuxième signal ECG et sa VFC, le deuxième signal ECG est obtenu par ajout de deux faux battements au premier signal ECG.

Les FIGURES 6(c) et 6(d) illustrent respectivement les spectres de VFC obtenues par réalisation d'une analyse fréquentielle typique de la VFC du premier signal ECG et ceux pour le deuxième signal ECG.

La FIGURE 7(a) illustre les résultats d'un exemple d'un calcul du paramètre NS.

La FIGURE 7(b) illustre les résultats d'un exemple d'un calcul des paramètres NS($t_X$), NSR($t_X$) et RC($t_X$) pendant une phase de repos, une phase d'activité sportive et une phase de récupération.

Les dessins joints sont donnés à titre d'exemples et ne sont pas limitatifs de l'invention. Ces dessins sont des représentations schématiques et ne sont pas nécessairement à l'échelle de l'application pratique.

DESCRIPTION DÉTAILLÉE DE L'INVENTION

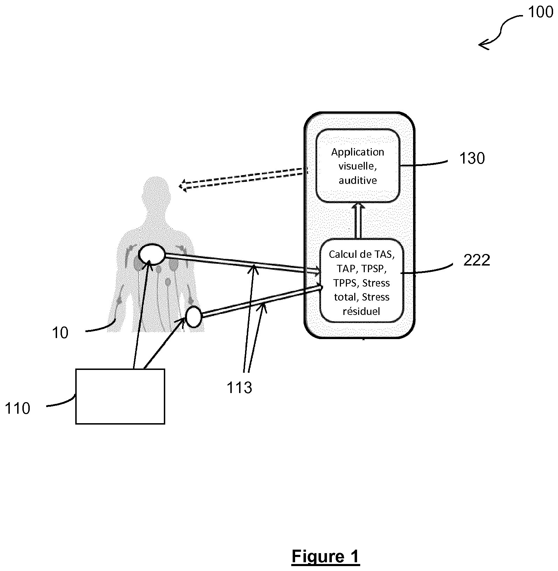

La **figure 1** illustre un système de surveillance 100 du système nerveux autonome (SNA) d'un sujet physique 10 selon un mode de réalisation de l'invention.

Le système de surveillance 100, comprend un module de traitement des données 222 et un module d'affichage des données 130. Il est configuré pour analyser un signal physiologique reçu d'un capteur de signaux physiologiques 110, typiquement mais non limitativement un électrocardiogramme (ECG).

Le capteur de signaux physiologiques 110 est configuré pour détecter des battements cardiaques de la personne physique 10 pendant une certaine durée et envoyer ainsi un signal physiologique au module de traitement des données 222.

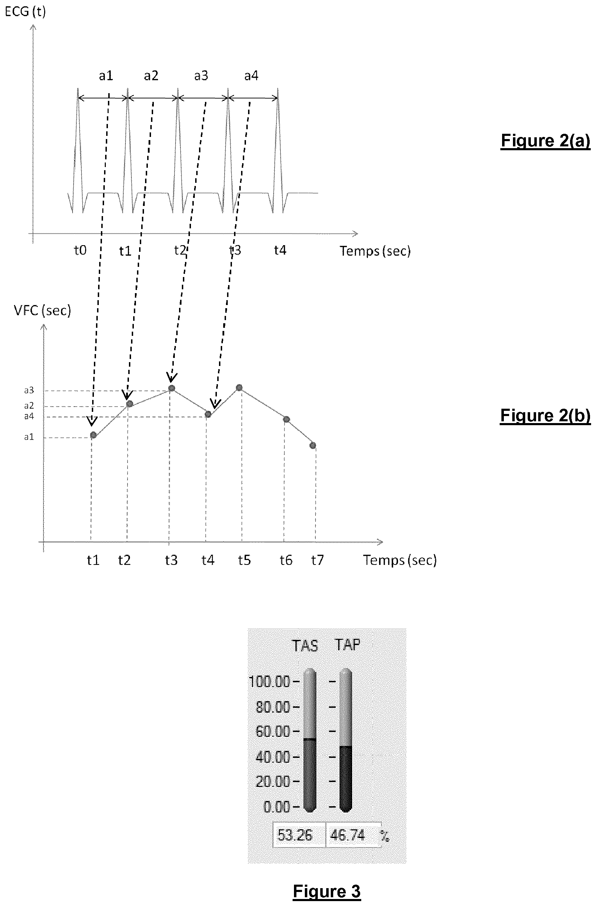

Le capteur de signaux physiologiques 110 est de préférence un capteur électrocardiogramme (ECG) configuré pour générer un signal physiologique ECG tel qu'illustré en **figure 2(a)** et envoyer ce signal ECG au module de traitement des données 222. Ce capteur ECG 110 est apte à être porté par la personne 10 par exemple sur son thorax ou/et son poignet.

Dans un mode de réalisation alternatif, le capteur de signaux physiologiques 110 porté par la personne 10 peut être un des dispositifs suivants : une ceinture thoracique, un dispositif photopléthysmographique installé dans un bracelet ou dans une montre.

L'invention n'est pas limitée à des méthodes d'acquisition du signal physiologique. Par exemple, un signal indiquant des battements cardiaques mesurés par des dispositifs optiques peuvent également être appliqués comme source de données de calculs de l'invention.

Le module de traitement des données 222 est couplé, par une communication filaire ou sans fil tel que par ondes radio, au capteur de signaux physiologiques 110. Le module de traitement des données 222 est configuré pour exécuter un procédé d'analyse de préférence dans le domaine temporel à partir d'un signal physiologique tel qu'un signal ECG reçu du capteur ECG 110. Le module de traitement des données 222 peut être réalisé soit dans un dispositif porté par la personne 10 soit dans un appareil à distance de la personne 10.

Selon un mode de réalisation, le module de traitement des données 222 est solidaire du capteur de signaux physiologiques 110 porté par la personne 10. Le module de traitement des données 222 et le capteur de signaux physiologiques 110 peuvent ainsi être intégrés dans une montre ou un bracelet par exemple.

Selon un autre mode de réalisation, le module de traitement des données 222 est un dispositif apte à être porté par la personne 10, qui est mécaniquement distinct du capteur de signaux physiologiques 110. Le module de traitement des données 222 et le capteur 110 sont distants, tout en étant couplés en termes de communication.

Selon un mode de réalisation alternatif, le module de traitement des données 222 est installé dans un appareil électronique tel qu'un téléphone portable, une tablette, un ordinateur, etc.

Selon un autre mode de réalisation alternatif, le module de traitement des données 222 est installé dans un serveur de données équipé d'un moyen de calcul est de traitement de données.

Dans un mode de réalisation préférentiel, le module de traitement des données 222 lui-même ou l'appareil électronique ou le serveur de données ci-dessus est muni d'un récepteur configuré pour recevoir le signal physiologique envoyé par le capteur ECG 110.

Dans le mode de réalisation où le module de traitement des données 222 est incorporé au serveur de données, le signal physiologique peut être transmis directement du capteur ECG 110 au serveur de données, ou par un relais tel qu'un appareil électronique, par exemple un téléphone portable, une tablette, un ordinateur, au serveur de données.

De manière avantageuse, le module de traitement des données 222 est réalisé sous forme d'un logiciel.

Dans le présent mode de réalisation, un module de communication filaire 113 est utilisé pour réaliser la communication entre le capteur ECG 110 et le module de traitement des données 222.

L'invention n'est pas limitée à des moyens de communication utilisés pour la réalisation de la communication filaire ou sans fil entre le module de traitement des données 222 et le capteur ECG 110.

Ledit procédé d'analyse effectué par le module de traitement des données 222 comprend un calcul d'intervalles RR et des calculs des paramètres TAS, TAP, TPSP, TPPS qui seront décrits ultérieurement.

La **figure 2(a)** montre un signal ECG comprenant N sommets signifiant respectivement un battement cardiaque de la personne 10 mesuré à un instant $t_0$, $t_1$ à $t_{N-1}$ par le capteur 110.

La **figure 2(b)** montre une variabilité de la fréquence cardiaque VFC illustrée par N-1 intervalles RR $a_1$, $a_2$ à $a_{N-1}$ correspondant respectivement aux instants $t_1$, $t_2$ à $t_{N-1}$

Chaque valeur $a_n$ d'un intervalle RR est la durée (typiquement en secondes) qui s'écoule entre deux sommets adjacents du signal ECG, ces deux sommets correspondant aux deux battements cardiaques successifs mesurés aux instants $t_{n-1}$ et $t_n$. La valeur $a_n$ est représentée dans l'équation (1) ci-dessous :

$$a_n = t_n - t_{n-1} \qquad \text{Eq. (1)}$$

Par exemple, comme indiqué en **figure 2(a),** la valeur $a_1$ correspond à l'intervalle de temps RR entre les deux battements cardiaques détectés aux deux instants $t_0$ et $t_1$.

La valeur du premier intervalle est notée $a_1$ et le nombre n des intervalles RR est égal à N-1.

Le module de traitement des données 222 effectue, à partir des N-1 intervalles RR $a_1$, $a_2$ à $a_{N-1}$ de la VFC, des calculs des paramètres TAS et TAP présentant respectivement l'activité du système sympathique et celle du système parasympathique de la personne 10 surveillée durant une durée d'échantillonnage TE comprenant N battements cardiaques du signal ECG.

Les calculs des paramètres TAS et TAP utilisent un phénomène physiologique : une durée d'un intervalle RR qui diminue entre deux battements cardiaques successifs indique un rythme cardiaque accéléré par le système sympathique alors qu'une durée d'un intervalle RR qui augmente entre deux battements successifs indique un rythme cardiaque ralenti par le système parasympathique.

Le paramètre TAS est configuré pour représenter le taux d'activité sympathique :

$$TAS = 100 \cdot \frac{\sum\limits_{n=1}^{N-1} (t_n - t_{n-1}) \text{ si } (a_n \leq a_{n-1})}{\sum\limits_{n=1}^{N-1} (t_n - t_{n-1})} = 100 \cdot \frac{\sum\limits_{n=1}^{N-1} a_n \text{ si } (a_n \leq a_{n-1})}{\sum\limits_{n=1}^{N-1} a_n} \qquad \text{Eq. (2)}$$

Le paramètre TAS est calculé, à partir des N-1 intervalles RR dérivés du signal ECG comprenant N battements cardiaques. Pour obtenir ce paramètre, on accumule (c'est-à-dire que l'on somme) les valeurs $a_n$ si un segment entre deux valeurs $a_n$ et $a_{n-1}$ est descendant comme illustré en **figure 2(b)** ; c'est-à-dire on accumule des valeurs $a_n$ dont chacune est inférieure à la valeur immédiatement précédente $a_{n-1}$. La somme des valeurs $a_n$ correspondant aux segments descendants est ensuite divisée par la somme de toutes les valeurs $a_1$, $a_2$ à $a_{N-1}$.

Le paramètre TAP est configuré pour représenter le taux d'activité parasympathique :

$$TAP = 100 \cdot \frac{\sum\limits_{n=1}^{N-1} (t_n - t_{n-1}) \text{ si } (a_n \geq a_{n-1})}{\sum\limits_{n=1}^{N-1} (t_n - t_{n-1})} = 100 \cdot \frac{\sum\limits_{n=1}^{N-1} a_n \text{ si } (a_n \geq a_{n-1})}{\sum\limits_{n=1}^{N-1} a_n} \qquad \text{Eq. (3)}$$

Le paramètre TAP est calculé, à partir des N-1 intervalles RR de la VFC dérivée du signal ECG comprenant N battements cardiaques, de manière à accumuler des valeurs $a_n$ si un segment entre deux valeurs $a_n$ et $a_{n-1}$ est montant comme illustré en **figure 2(b)** ; c'est-à-dire on accumule des valeurs $a_n$ dont chacune est supérieure à la valeur immédiatement précédente $a_{n-1}$. La somme des valeurs $a_n$ correspondant aux segments montants est ensuite divisée par la somme de toutes les valeurs $a_1$, $a_2$ à $a_{N-1}$.

La **figure 3** montre des résultats des calculs des paramètres TAS et TAP sur une durée d'échantillonnage TE de 100 battements cardiaques (N=100). La représentation visuelle de la figure 3, présente pour avantage de pouvoir comparer très facilement les activités respectives des systèmes sympathique et parasympathique. Dans cet exemple, le calcul des paramètres TAS et TAP permet d'identifier très aisément que le système SNA de la personne 10 est dans un équilibre relatif et imparfait car le système sympathique est légèrement plus actif que celui parasympathique. Par ailleurs, cette représentation varie en temps réel afin de suivre en temps réel l'évolution des activités respectives des systèmes sympathique et parasympathique. Ce suivi en temps réel permet par exemple au patient de contrôler son SNA en effectuant des actions appropriées, notamment pour augmenter l'activité de son système parasympathique.

Au niveau de la fiabilité du calcul d'un point de vue mathématique, c'est-à-dire dans une condition idéale où le signal ECG est bien capturé par le capteur ECG 110, les calculs des paramètres TAS et TAP sont, par rapport à ceux des méthodes connues d'analyse fréquentielle, beaucoup plus précis. Le nombre N de battements peut être réduit à un nombre compris entre 20 et 40.

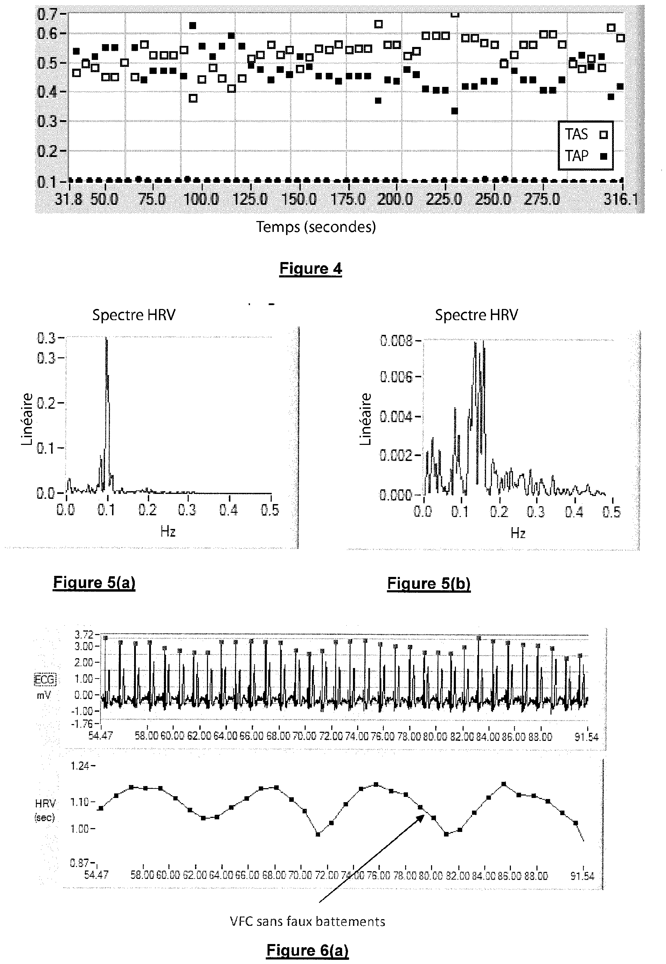

Des comparaisons des deux types de calculs (méthode de l'invention et méthodes connues d'analyse fréquentielle) seront fournies dans les **figures 5(a), 5(b)** et **6(a)** à **6(d)** illustrées ultérieurement.

Néanmoins, par exemple lorsque le signal ECG de la personne 10 est capturé lors d'une activité sportive, le nombre N de battements est compris de préférence entre 200 et 350 (c'est-à-dire la durée d'enregistrement des battements d'un signal ECG est environ de trois minutes), car en pratique, 200 à 350 battements successifs permettent de refléter un état physiologique pertinent.

En effet, les calculs proposés par l'invention font appel à des implémentations mathématiques simples, ce qui permet d'obtenir en temps réel un résultat de calcul, notamment dans un mode de réalisation où le nombre N de battements cardiaques est inférieur à, par exemple, 1000.

Ainsi, deux paramètres $TAS(t_X)$ et $TAP(t_X)$ montrant respectivement l'activité du système sympathique et celle du système parasympathique, peuvent être calculés, en temps réel, en utilisant par exemple un certain nombre de derniers battements cardiaques mesurés par le capteur ECG 110.

De manière préférentielle mais non limitative, une fenêtre glissante d'échantillonnage peut être utilisée pour échantillonner W battements cardiaques qui seront utilisés pour calculer les paramètres $TAS(t_X)$ et $TAP(t_X)$ d'un signal ECG. L'invention n'est pourtant pas limitée à des méthodes de sélection ou d'échantillonnage des battements cardiaques utilisés pour les calculs des paramètres $TAS(t_X)$ et $TAP(t_X)$.

Avec une fenêtre glissante dont le nombre d'échantillons est W, le module de traitement des données 222 peut effectuer, à chaque instant tel un nouveau battement cardiaque mesuré à l'instant $t_X$ du signal ECG, des calculs des paramètres $TAS(t_X)$ et $TAP(t_X)$ pour obtenir en temps réel des résultats de calculs qui montrent l'activité du système sympathique et celle du système parasympathique entre deux battements cardiaques (i.e. le $(X-W+1)^{ème}$ et le $x^{ème}$

battements cardiaques) respectivement aux instants $t_{X-W+1}$ et $t_X$ du signal ECG.

Le nombre des intervalles RR entre les deux battements cardiaques aux instants $t_{X-W+1}$ et $t_X$ est donc W-1.

De manière préférentielle mais non limitative, les calculs des paramètres TAS($t_X$) et TAP($t_X$) sont effectués après le $W^{ème}$ battement cardiaque du signal ECG. Par exemple, pour obtenir un résultat reflétant les activités des systèmes sympathique et parasympathique, le nombre d'échantillons W est compris par exemple entre 10 et 30.

Le calcul du paramètre TAS($t_X$) est représenté dans l'équation (4) ci-dessous :

$$TAS(t_X) = 100 \cdot \frac{\sum\limits_{k=X-W+2}^{X} (t_k - t_{k-1}) \ si \ (a_k \leq a_{k-1})}{\sum\limits_{k=X-W+2}^{X} (t_k - t_{k-1})} \qquad \text{Eq. (4),}$$

dans laquelle $a_k = t_k - t_{k-1}$

De manière proche de celle du calcul du paramètre TAS, le paramètre TAS($t_X$) est calculé, à partir des W-1 intervalles RR de la VFC dérivée d'une durée d'échantillonnage TE du signal ECG comprenant W battements cardiaques entre les deux battements cardiaques respectivement mesurés aux instants $t_{X-W+1}$ et $t_X$ du signal ECG, de manière à sommer les valeurs $a_k$ si un segment entre deux valeurs $a_k$ et $a_{k-1}$ est descendant ; c'est-à-dire on accumule les valeurs $a_k$ dont chacune est inférieure à la valeur immédiatement précédente $a_{k-1}$. La somme des valeurs $a_n$ correspondant aux segments descendants est ensuite divisée par la somme de toutes les W-1 valeurs $a_{X-W+2}$, $a_{X-W+3}$ à $a_X$.

Le calcul du paramètre TAP($t_X$) suit l'équation (5) ci-dessous :

$$TAP(t_X) = 100 \cdot \frac{\sum\limits_{k=X-W+2}^{X} (t_k - t_{k-1}) \ si \ (a_k \geq a_{k-1})}{\sum\limits_{k=X-W+2}^{X} (t_k - t_{k-1})} \qquad \text{Eq. (5)}$$

De manière proche de celle du calcul du paramètre TAP, le paramètre TAP($t_X$) est calculé, à partir des W-1 intervalles RR de la VFC dérivée d'une durée d'échantillonnage TE comprenant W battements cardiaques entre les deux battements cardiaques respectivement mesurés aux instants $t_{X-W+1}$ et $t_X$ du signal ECG, de manière à accumuler des valeurs $a_k$ si un segment entre deux valeurs $a_k$ et $a_{k-1}$ est montant ; c'est-à-dire on accumule des valeurs $a_k$ dont chacune est supérieure à la valeur immédiatement précédente $a_{k-1}$. La somme des valeurs $a_n$ correspondant aux segments montants est ensuite divisée par la somme de toutes les W-1 valeurs $a_{X-W+2}$, $a_{X-W+3}$ à $a_X$.

La **figure 4** illustre les paramètres TAS($t_X$) et TAP($t_X$) obtenus en temps réel par un calcul basé sur 30 échantillons de la fenêtre glissante à chaque instant $t_X$ (W = 30). Comme illustré en **figure 4,** les états des systèmes sympathique et parasympathique de la personne 10 sont calculés en temps réel et montrés au fur et à mesure de la durée d'échantillonnage TE entre l'instant de départ à la 32$^{ème}$ seconde et l'instant de fin à la 316$^{ème}$ seconde.

Selon un mode de réalisation avantageux mais optionnel, le module de traitement des données 222 effectue en outre le calcul des paramètres désignés TPSP et TPPS.

Le paramètre TPSP est configuré pour représenter le taux de pollution du système sympathique vers le système parasympathique qui indique probablement une mauvaise qualité de respiration de la personne 10 à cause du stress perçu ou d'un effort physique Le calcul du paramètre TPSP est calculé selon l'équation (6) ci-dessous :

$$TPSP = 100 \cdot \frac{\sum\limits_{n=1}^{N-1} (t_n - t_{n-1}) \ si \ (a_n < a_{n-1} \ et \ a_{n-1} > a_{n-2} \ et \ a_{n+1} > a_n)}{\sum\limits_{n=1}^{N-1} (t_n - t_{n-1})} \qquad \text{Eq. (6)}$$

Le paramètre TPSP est calculé, à partir des N-1 intervalles RR de la VFC dérivée du signal ECG comprenant N battements cardiaques, de manière à accumuler des valeurs $a_n$ si un segment entre deux valeurs $a_n$ et $a_{n-1}$ est descendant et entre deux segments adjacents montants dont l'un précède ledit segment et l'autre suit ledit segment.

Autrement dit, ce taux de pollution TPSP prend en compte un segment descendant qui perturbe la tendance monotone croissante, également désignée tendance croissante, formée par deux segments montants dont l'un précède ledit segment descendant et l'autre suit ledit segment descendant.

La somme des valeurs $a_n$ satisfaisant les conditions ci-dessus est ensuite divisée par la somme de toutes les valeurs $a_1$, $a_2$ à $a_{N-1}$.

Le paramètre TPPS est configuré pour représenter le taux de pollution du système parasympathique vers le système sympathique qui indique probablement une mauvaise qualité de respiration de la personne 10 lors d'un repos ou de la récupération après un effort physique. Le calcul du paramètre TPPS est représenté dans l'équation (7) ci-dessous :

$$TPPS = 100 \cdot \frac{\sum_{n=1}^{N-1} (t_n - t_{n-1}) \ si \ (a_n > a_{n-1} \ et \ a_{n-1} < a_{n-2} \ et \ a_{n+1} < a_n)}{\sum_{n=1}^{N-1} (t_n - t_{n-1})} \qquad Eq. (7)$$

Le paramètre TPPS est calculé, à partir des N-1 intervalles RR de la VFC dérivée du signal ECG comprenant N battements cardiaques, de manière à accumuler des valeurs $a_n$ si un segment entre deux valeurs $a_n$ et $a_{n-1}$ est montant et entre deux segments adjacents descendants dont l'un précède ledit segment et l'autre suit ledit segment.

Autrement dit, ce taux de pollution TPPS prend en compte un segment montant qui perturbe la tendance monotone descendante, également désignée tendance décroissante, formée par deux segments descendants dont l'un précède ledit segment montant et l'autre suit ledit segment montant.

La somme des valeurs $a_n$ satisfaisant les conditions ci-dessus est ensuite divisée par la somme de toutes les valeurs $a_1$, $a_2$ à $a_{N-1}$.

Dans un autre mode de réalisation, une estimation simplifiée des taux de pollutions TPSP et TPPS peut être obtenue en calculant le nombre d'occurrences où les tendances monotones (croissante ou décroissante) de la VFC par rapport au nombre des intervalles RR qui est N-1.

Deux paramètres $\overline{TPSP}$ et $\overline{TPPS}$, une estimation simplifiée pour les paramètres TPSP et TPPS respectivement, sont représentés dans les équations (8) et (9) ci-dessous:

$$\overline{TPSP} = 100 \cdot \frac{\sum_{n=1}^{N-1} 1 \ si \ (a_n < a_{n-1} \ et \ a_{n-1} > a_{n-2} \ et \ a_{n+1} > a_n)}{N-1} \qquad Eq. (8)$$

$$\overline{TPPS} = 100 \cdot \frac{\sum_{n=1}^{N-1} 1 \ si \ (a_n > a_{n-1} \ et \ a_{n-1} < a_{n-2} \ et \ a_{n+1} < a_n)}{N-1} \qquad Eq. (9)$$

Les valeurs des paramètres $\overline{TPSP}$ et $\overline{TPPS}$ sont les versions simplifiées des paramètres TPSP et TPPS au niveau de calculs mathématiques. Elles sont considérées comme des estimations approximées qui sont en pratique souvent proches des valeurs des paramètres TPSP et TPPS.

Comme pour les paramètres TAS et TAP, les calculs ci-dessus pour obtenir les paramètres TPSP et TPPS sont des implémentations mathématiques simples, ce qui permet ainsi d'obtenir des résultats en temps réel, notamment dans un mode de réalisation où le nombre N de battements cardiaques est inférieur à, par exemple, 1000.

Ainsi, comme pour les calculs des paramètres $TAS(t_X)$ et $TAP(t_X)$, le module de traitement des données 222 effectue, à chaque instant tel qu'un nouveau battement cardiaque mesuré à l'instant $t_X$ du signal ECG, des calculs des paramètres $TPSP(t_X)$ et $TPPS(t_X)$ utilisant W battements cardiaques entre deux battements cardiaques (i.e. le (X-W+1)ème et le Xème battements cardiaques) respectivement aux instants $t_{X-W+1}$ et $t_X$ d'un signal ECG.

Ces W battements cardiaques peuvent être obtenus par utilisation d'une fenêtre glissante. L'invention n'est pourtant pas limitée à des méthodes de sélection ou d'échantillonnage des battements cardiaques utilisés pour les calculs des paramètres $TPSP(t_X)$ et $TPPS(t_X)$.

De manière préférentielle mais non limitative, les calcul des paramètres $TAS(t_X)$ et $TAP(t_X)$ sont effectués après le Wème battement cardiaque du signal ECG. De plus, le nombre d'intervalles RR de la VFC entre les deux battements cardiaques mesurés aux instants $t_{X-W+1}$ et $t_X$ est W-1.

Le calcul du paramètre $TPSP(t_X)$ est représenté dans l'équation (10) ci-dessous

$$TPSP(t_X) = 100 \cdot \frac{\sum_{k=X-W+2}^{X} (t_k - t_{k-1}) \; si \; (a_k < a_{k-1} \; et \; a_{k-1} > a_{k-2} \; et \; a_{k+1} > a_k)}{\sum_{k=X-W+2}^{X} (t_k - t_{k-1})} \qquad \text{Eq. (10)}$$

De manière proche de celle du calcul du paramètre TPSP, le paramètre TPSP($T_X$) est calculé, à partir des W-1 intervalles RR échantillonnés, de manière à accumuler des valeurs $a_k$ si un segment donné entre deux valeurs $a_k$ et $a_{k-1}$ est descendant et perturbe donc la tendance monotone croissante formée par les deux segments montants et adjacents audit segment donné dont l'un précède ledit segment descendant et l'autre suit immédiatement ledit segment descendant.

La somme des valeurs $a_k$ satisfaisant les conditions ci-dessus est ensuite divisée par la somme de toutes les W-1 valeurs $a_{X-W+2}$, $a_{X-W+3}$ ..., à $a_X$.

Le calcul du paramètre TPPS($t_X$) est représenté dans l'équation (11) ci-dessous

$$TPPS(t_X) = 100 \cdot \frac{\sum_{k=X-W+2}^{X} (t_k - t_{k-1}) \; si \; (a_k > a_{k-1} \; et \; a_{k-1} < a_{k-2} \; et \; a_{k+1} < a_k)}{\sum_{k=X-W+2}^{X} (t_k - t_{k-1})} \qquad \text{Eq. (11)}$$

De manière très similaire à celle du calcul du paramètre TPPS, le paramètre TPPS($T_X$) est calculé, à partir des W-1 intervalles RR échantillonnés, de manière à accumuler des valeurs $a_k$ si un segment donné entre deux valeurs $a_k$ et $a_{k-1}$ est montant et perturbe donc la tendance monotone décroissante formée par les deux segments descendants et adjacents audit segment donné dont l'un précède ledit segment montant et l'autre suit ledit segment montant.

La somme des valeurs $a_k$ satisfaisant les conditions ci-dessus est ensuite divisée par la somme de toutes les W-1 valeurs $a_{X-W+2}$, $a_{X-W+3}$ ..., $a_X$.

Les paramètres TPSP et TPPS représentent la qualité de la respiration (TPSP pour l'inspiration et TPPS pour l'expiration) de la personne 10. Quand la personne 10 est en état de repos en pratiquant une respiration cohérente, les valeurs des paramètres TPSP et TPPS sont inférieures à 1% et peuvent être très proches de 0%. Dans un autre cas où la personne 10 est toujours en état de repos mais en respirant normalement (sans cohérence cardiaque), les valeurs des paramètres TPSP et TPPS sont d'environ de 10%.

Le module d'affichage des données 130 est couplé, par une communication filaire ou sans fil tel que par ondes radio, au module de traitement des données 222. Ce module d'affichage des données 130 est configuré pour recevoir au moins une partie des résultats du procédé d'analyse effectué par le module de traitement des données 222 tels que les paramètres TAS, TAP, TPSP, TPPS, TAS($t_X$), TAP($t_X$), TPSP($t_X$), TPPS($t_X$) et en donner une représentation visuelle sur un écran d'affichage. Par exemple, les résultats des calculs des paramètres TAS et TAP comme illustrés en **figures 3 et 4** sont visuellement affichés de manière graphique par le module d'affichage des données 130. Des résultats des calculs des paramètres TAS, TAP, TPSP, TPPS, TAS($t_X$), TAP($t_X$), TPSP($t_X$), TPPS($t_X$) peuvent être affichés de manière graphique ou auditive.

Selon un mode de réalisation, le module d'affichage des données 130 est incorporé à un appareil électronique tel qu'un téléphone portable, une tablette, un ordinateur, une montre, etc.

L'invention n'est pas limitée à des moyens de communication utilisés pour la réalisation de la communication filaire ou sans fil entre le module d'affichage des données 130 et le module de traitement des données 222 ni à l'implémentation du module d'affichage des données 130.

D'autres exemples de représentation visuelle affichables sur un écran sont donnés ci-dessous :

- Objet (voiture, avion), personnage humain ou animal dont le comportement ou l'animation varie en fonction du ou des paramètres calculés.
- Figures géométriques dont les dimensions et/ou la couleur varient en fonction du ou des paramètres calculés (par exemple cercles, ellipses, carrés, triangles).

Les calculs des paramètres TAS, TAP, TPSP, TPPS, TAS($t_X$), TAP($t_X$), TPSP($t_X$), TPPS($t_X$) du procédé d'analyse selon l'invention, effectués par le module de traitement des données 222, sont des analyses temporelles à partir de la VFC dérivée d'un signal ECG.

De plus, les paramètres tels que ci-dessus permettent de mesurer et d'indiquer le niveau énergétique du corps de la personne 10 en état de repos ou/et en activité par exemple pendant une activité sportive.

Le procédé d'analyse temporelle de l'invention présente ainsi plusieurs avantages ci-dessous :

1) Surveillance des systèmes sympathique et parasympathique de manière indépendante et simultanée :

Comme mentionné ci-dessus, le procédé de l'invention divise les valeurs $a_1$, $a_2$, à $a_{N-1}$ des intervalles RR en deux groupes.

Plus précisément, le paramètre TAS, reflétant l'activité du système sympathique, est calculé de manière à sommer seulement les valeurs $a_n$ correspondant aux segments descendants comme explicité ci-dessus alors que le paramètre TAP, reflétant l'activité du système parasympathique, est calculé de manière à sommer seulement les valeurs $a_n$ correspondant aux segments montants. Les deux branches parasympathique et sympathique peuvent être ainsi surveillées indépendamment en temps réel, ce qui permet à une personne ou son médecin de vérifier en temps réel le niveau de stress perçu (par exemple lorsque le paramètre TAS est supérieur au paramètre TAP) et de déterminer des remèdes adaptés et réalisés de préférence sur place pour diminuer la valeur du paramètre TAS. Le résultat des remèdes appliqués peuvent être également vérifiés sur place et en temps réel. Le médecin ou l'utilisateur peuvent alors rétroagir sur les remèdes ou actions à effectuer pour ramener l'activité du SNA à la valeur souhaitée ou ramener l'équilibre entre systèmes sympathique et parasympathique à l'équilibre souhaité.

Les paramètres TPSP et TPPS reprennent ces caractéristiques et permettent donc également de montrer individuellement, durant la même période de surveillance, le niveau d'interférence entre les deux branches sympathique et parasympathique du SNA, ce qui permet ainsi à une personne ou son médecin de vérifier en temps réel la qualité de sa respiration.

Plus la valeur des paramètres TPSP et TPPS est grande, moins la qualité de la respiration est bonne. Dans le cas d'une respiration normale et inconsciente, les valeurs de paramètres TPSP et TPPS sont d'environ de 10%. Dans le cas d'une respiration cohérente et symétrique, par exemple 5 secondes d'inspiration et 5 secondes d'expiration, les valeurs des paramètres TPSP et TPPS sont sensiblement égales à zéro.

En revanche, les méthodes d'analyse fréquentielle appliquant des calculs tels que la FFT sur la VFC ne peuvent pas séparer les données concernant la branche sympathique de celles concernant la branche parasympathique. Autrement dit, les données concernant les systèmes sympathique et parasympathique sont mélangées et de manière aléatoire. La séparation des données de ces deux systèmes n'est alors pas possible.

Une personne ou son médecin ne peut alors pas déterminer, à partir des calculs obtenus par les méthodes d'analyse fréquentielle, l'activité de la branche sympathique indépendamment de celle de la branche parasympathique et inversement.

Si une personne inspire pendant 5 secondes et expire pendant les 5 secondes suivantes, on peut considérer qu'il respire de façon cohérente symétrique.

La **figure 5(a)** montre un spectre de la VFC qui comporte un seul sommet à 0.1Hz et correspond à une période de respiration de 10 secondes (5 secondes d'inspiration et 5 secondes d'expiration). Ainsi, il n'est pas possible de séparer les données des deux branches sympathique et parasympathique à partir d'un tel spectre. En effet, la seule information valide obtenue à partir du spectre est la fréquence respiratoire dont le sommet est à 0.1Hz.

Dans un deuxième exemple, une personne respire de manière inconsciente (aléatoire) dans un état de repos. La **figure 5(b)** montre un spectre de la VFC selon le deuxième exemple.

On remarque qu'une distribution spectrale aléatoire change à chaque fois en raison du changement de l'activité réalisée par la personne et/ou du changement du rythme respiratoire de la personne. La distribution spectrale de la VFC représente donc la fréquence respiratoire comportant les données des deux branches du SNA déjà cumulées et mélangées de manière inséparable dans le domaine temporel.

Il n'est alors plus possible de discriminer chaque composante à partir de la distribution spectrale de la VFC. Autrement dit, il est alors impossible d'extraire les informations souhaitées à partir de la distribution spectrale de la VFC comportant les données inséparablement mélangées des deux branches du SNA.

2) Suivi en temps réel :

Les calculs de l'analyse temporelle tels que ci-dessus sont des implémentations mathématiques simples.

Par rapport aux méthodes d'analyse fréquentielle impliquant des calculs exigeant une complexité considérablement plus élevée comme la FFT, la méthode CDM, etc, les calculs des paramètres TAS, TAP, TPSP, TPPS, TAS($t_X$), TAP($t_X$), TPSP($t_X$), TPPS($t_X$) consomment moins de temps d'exécution et permettent donc de surveiller en temps réel les activités des systèmes sympathique et parasympathique. Il est alors possible de rétroagir en temps réel pour contrôler l'activité du SNA.

Dans un exemple de réalisation des méthodes d'analyse fréquentielle en temps réel où W (le nombre d'échantillons de la fenêtre glissante FFT) est 20 et le rythme cardiaque est de 60 battements/minute, la précision spectrale est

$$(\text{Hertz}) = \frac{1}{20} \text{ Hz},$$

de 0.05Hz et il en résulte qu'il faut analyser et séparer un spectre qui s'étale entre 0 et 0.5Hz avec seulement 10 points de FFT.

Ainsi, pour obtenir une précision raisonnable par exemple de 0.005Hz, W devrait être 200, avec les méthodes basées sur une analyse fréquentielle, il est nécessaire d'avoir les données de 200 battements cardiaques. La technique d'insertion des zéros, de l'anglais « zéro padding » peut éventuellement augmenter la résolution spectrale mais n'augmente pas la précision de l'analyse fréquentielle. Il faut donc un temps d'exécution de plus de 3 minutes avec un rythme cardiaque moyen de par exemple 60 battements/minute pour avoir un premier résultat ; autrement dit, le résultat de calculs ne peut être facilement obtenu en temps réel.

3) Précision améliorée grâce au meilleur rapport signal sur bruit et une sensibilité réduite aux faux battements cardiaques.

La sensibilité aux faux battements et/ou aux diverses arythmies du procédé d'analyse temporelle de l'invention est faible en raison d'une dépendance linéaire entre l'erreur et le résultat.

Dans un exemple utilisant 300 battements cardiaques dont deux sont des faux battements, au niveau des calculs

$$\frac{2}{300} = 0.0066\%$$

des paramètres TAS et TAP de l'invention, le taux d'erreur est d'environ et est donc négligeable. A l'inverse, en mettant en oeuvre l'analyse fréquentielle et en prenant le même exemple, deux faux battements conduisent à un effet bien supérieur comme montré par la suite en référence aux **figures 6(a)** à **6(d).**

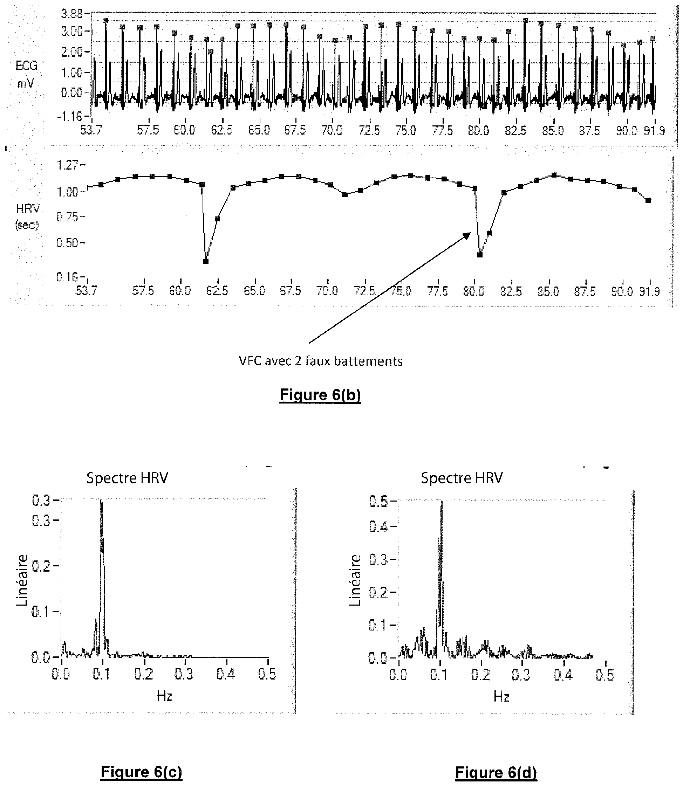

La **figure 6(a)** montre une partie d'un premier signal ECG et sa VFC, le premier signal ECG comprenant 300 battements sans ajout de faux battements cardiaques. La **figure 6(b)** montre une partie d'un deuxième signal ECG et sa VFC, le deuxième signal ECG étant obtenu par ajout de deux faux battements respectivement aux instants t = 62 sec et t = 80.5 sec au premier signal ECG.

Les **figures 6(c)** et **6(d)** illustrent respectivement les spectres de VFC obtenues par réalisation d'une analyse fréquentielle conventionnelle de la VFC du premier signal ECG (sans faux battement) et ceux pour le deuxième signal ECG (avec deux faux battements).

Le spectre montré en **figure 6(d)** est considérablement affecté par les bruits résultant des deux faux battements. Les deux faux battements pourraient éventuellement être enlevés manuellement après la fin de l'acquisition d'un signal ECG et avant l'analyse spectrale de la VFC, mais dans ce cas toute notion de temps réel disparaît. De plus, en un cas d'arythmie, les spectres de VFC seraient encore plus sérieusement affectés.

Ainsi, il est clair que le procédé d'analyse temporelle de l'invention est, par rapport aux méthodes d'analyse fréquentielle, moins sensible aux bruits.

4) Procédé non-invasif et apte à être utilisé en milieu sportif

Les calculs sont basés sur des intervalles RR et n'ont pas besoin d'autres types de données. Les intervalles RR peuvent être mesurés par un capteur ECG 110 de manière non invasive et dans un milieu imposant des contraintes tel qu'un milieu sportif.

Les paramètres TAS, TAP, TPSP et TPPS de l'invention montrent ainsi efficacement l'activité du système sympathique et celle du système parasympathique et permettent alors aux chercheurs et médecins de disposer d'une représentation précise et fiable du SNA, ce qui leur permet ainsi d'accroitre la pertinence de leurs diagnostics et de réduire le temps et donc le coût d'acquisition et d'obtention de la représentation. Un utilisateur peut par ailleurs vérifier son état de stress en visualisant les résultats desdits paramètres, et peut donc les corréler avec l'état émotionnel de la personne et/ou les activités physiques que la personne a effectuées.

Ces paramètres peuvent être appliqués à des calculs divers tels qu'un calcul du stress perçu et accumulé par une personne, un calcul montrant l'état émotionnel lié à une situation stressante, ou/et celui de l'énergie dépensée par un joueur lors d'une activité sportive, etc.

Une des applications est l'estimation du stress accumulé pendant une longue période. Par exemple, si une personne respire de manière cohérente et symétrique (i.e. 5 secondes d'inspiration suivies immédiatement de 5 secondes d'expiration, constituant une cohérence cardiaque), le résultat des calculs des paramètres TAS et TAP montre que le système SNA de la personne 10 est à l'équilibre. C'est-à-dire le TAS et le TAP sont respectivement 50% et le ratio TAS/TAP est donc égal à 1.

Néanmoins, si la personne subit un stress prolongé, par exemple plusieurs semaines, mois ou années, le résultat des calculs des paramètres TAS et TAP montre un déséquilibre (valeur du TAS supérieure à celle du TAP), et reflète cet état de stress.

Un paramètre NS configuré pour représenter le niveau de stress de la personne 10 est représenté dans l'équation (12) :

$$NS = 100 + TAS - TAP \qquad \text{Eq. (12)}$$

Cette estimation du niveau de stress peut être réalisée en temps réel, par exemple, à l'instant $t_X$ du signal ECG, en utilisant les paramètres $TAS(T_X)$ et $TAP(T_X)$ tels que ci-dessus. L'estimation du niveau de stress $NS(T_X)$ est représentée dans l'équation (13) :

$$NS(t_x) = 100 + TAS(t_x) - TAP(t_x) \qquad \text{Eq. (13)}$$

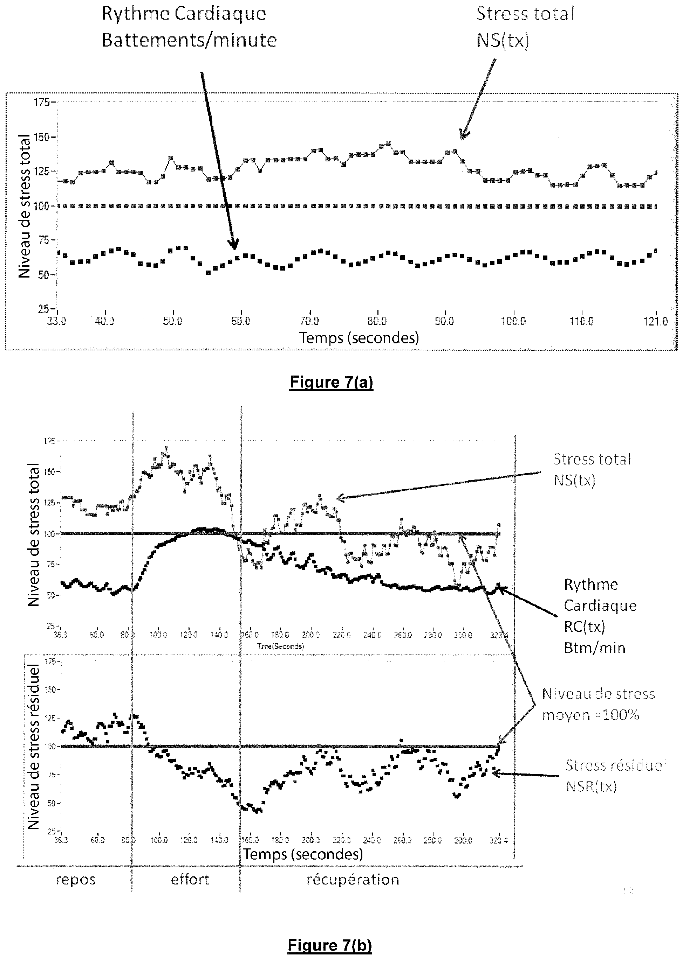

La **figure 7(a)** illustre les résultats d'un exemple d'un calcul du paramètre NS. Dans cet exemple, la personne 10 a subi un stress sur le long terme. Le niveau de stress NS est égal à 126.3%, ce qui montre un dysfonctionnement du système SNA avec une augmentation de l'activité sympathique et une baisse de l'activité parasympathique. Dans cet exemple, le ratio TAP/TAS est de 1.68.

En revanche, une valeur du paramètre NS inférieure à 100 (soit le ratio TAS/TAP étant inférieur à 1) montre que la personne ne subit pas de stress spécifique ou bien l'impact du stress est très limité par exemple en raison d'une excellente capacité de gestion du stress ou d'un environnement peu stressant.

Une valeur du paramètre NS égale à 100 montre que la personne n'a pas de problèmes particuliers de stress.

Selon un mode de réalisation avantageux, le module de traitement des données 222 est configuré pour générer un stimulus de respiration, de préférence symétrique, destiné à être fourni à la personne 10 pendant une durée T, de sorte à ajuster le niveau de stress de la personne 10. On désigne par respiration symétrique, une respiration au cours de laquelle l'inspiration est aussi longue que l'expiration.

Le stimulus de respiration comprend un stimulus visuel, ou/et un stimulus auditif ou/et olfactif. Un stimulus visuel comprend par exemple des images ou des vidéos relaxantes : paysage par beau temps tel qu'une plage ensoleillée. Un stimulus auditif comprend par exemple des bruits de la mer et des mouettes. Un stimulus olfactif comprend par exemple des odeurs de la mer.

Dans un mode de réalisation où le stimulus de respiration comprend une combinaison des stimuli visuels, auditifs ou/et olfactifs, ces stimuli visuels, auditifs et olfactifs sont de préférence cohérents entre eux et générés simultanément.

Le stimulus de respiration comprend une consigne respiratoire de sorte que la personne 10 respire de manière symétrique. Le stimulus de respiration dure un temps T qui est calculé, de préférence par le module de traitement des données 222, en fonction d'au moins le rythme cardiaque (RC). Selon un mode de réalisation préférentiel, la durée T est générée en utilisant un signal sinusoïdal d'une fréquence par exemple de 0.1Hz qui est obtenu en échantillonnant, avec une fréquence supérieure à 0.01Hz, le rythme cardiaque (RC). La consigne respiratoire de la durée T de 10 secondes comprend donc une inspiration de 5 secondes et une expiration de 5 secondes. Ainsi, le stimulus de respiration est fourni à la personne 10 pour que la personne 10 puisse réguler sa respiration en faisant une inspiration de 5 secondes et une expiration de 5 secondes.

Dans un mode de réalisation avantageux, le procédé de régulation comprend en outre une corrélation linéaire continue, réalisée préférentiellement par le module de traitement des données 222, en fonction d'au moins le stimulus de respiration et le RC en prenant un nombre d'échantillons de battements cardiaques compris entre 10 et 30 et, de préférence de 30. Le calcul de la corrélation linéaire permet d'estimer un niveau d'adaptation du système SNA de la personne 10 à la consigne du stimulus de respiration.

Dans un mode de réalisation avantageux, le RC est calculée en temps réel et la durée T est calculée et ajustée également en temps réel an appliquant une équation (14) dans laquelle le RC est le rythme cardiaque de la personne 10 :

$$T = 10 * \left(\frac{60}{RC}\right) \qquad \text{Eq. (14)}$$

Ainsi, la régulation du système SNA de la personne 10 est réalisée en temps réel, permettant donc à la personne 10 d'obtenir rapidement un maximum de confort.

De plus, une valeur de stress chronique SC est calculée en fonction de la valeur moyenne du paramètre $NS(t_X)$ sur ladite durée T du stimulus de respiration. De préférence, la valeur de stress chronique SC est égale à la valeur moyenne du paramètre $NS(t_X)$ sur ladite durée T du stimulus de respiration.

Une autre application de ces paramètres concerne l'estimation du stress résiduel d'une personne pendant une activité sportive. Un paramètre NSR configuré pour représenter le niveau de stress résiduel de la personne 10 est représenté dans l'équation (15) :

$$NSR = NS \cdot \frac{RC_{Repos}}{RC} \qquad \text{Eq. (15)}$$

Cette estimation du niveau de stress résiduel peut être réalisée en temps réel, par exemple, à l'instant $t_X$ du signal ECG. L'estimation du niveau de stress résiduel $NSR(T_X)$ est représentée dans l'équation (16) :

$$NSR(t_X) = NS(t_X) \cdot \frac{RC_{Repos}}{RC(t_X)} \qquad \text{Eq. (16)}$$

dans laquelle $RC_{Repos}$ est la fréquence cardiaque au repos, c'est-à-dire la personne 10 est inactive pendant au moins 20 secondes et de préférence 40 secondes, $RC(t_X)$ est la fréquence cardiaque à l'instant $t_X$.

La **figure 7(b)** illustre les résultats d'un exemple d'un calcul des paramètres $NS(t_X)$, $NSR(t_X)$ et $RC(t_X)$ pendant une phase de repos, une phase d'activité sportive et une phase de récupération.

Les paramètres TAS, TAP, TPSP, TPPS, les applications telles que NS, SC et NSR et leurs déclinaisons en temps réel, peuvent être utilisés dans des jeux vidéo et des simulateurs afin de montrer l'état du SNA et donc l'état émotionnel d'un joueur/patient. On peut ainsi détecter le niveau de stress, le niveau d'énergie de la personne.

Comme décrit précédemment, le module d'affichage des données 130 est configuré pour recevoir au moins une partie des résultats du procédé d'analyse effectué par le module de traitement des données 222 tels que les paramètres TAS, TAP, TPSP, TPPS, NS, SC, NSR, $TAS(t_X)$, $TAP(t_X)$, $TPSP(t_X)$, $TPPS(t_X)$, $NS(t_X)$, $NSR(t_X)$. Le module d'affichage des données 130 est configuré également pour afficher l'au moins une partie des résultats des paramètres ci-dessus, de manière visuelle (par exemple graphique) ou/et auditive.

Il faut noter qu'au niveau de fiabilité de calcul d'un point de vue mathématique, c'est-à-dire dans une condition idéal où le signal ECG est bien capturé par le capteur ECG 110, les calculs des paramètres et des applications proposés par l'invention sont, par rapport à ceux des méthodes connues d'analyse fréquentielle, beaucoup plus précis. Le nombre (N ou W) de battements peut être réduit à un nombre compris entre 20 et 40.

Une application possible consiste par exemple à prendre en compte l'activité de l'un ou plusieurs de ces paramètres relatifs au SNA pour modifier le comportement d'un avatar. Tel peut être le cas dans un jeu vidéo ou dans un programme médical ou de bien-être où l'avatar, personnage, animal ou objet animé verrait sa représentation et/ou son comportement et/ou ses facultés modifiés en fonction des paramètres relatifs au SNA.

Dans le cadre d'un jeu vidéo, un objectif serait d'accroître le réalisme du jeu et d'augmenter la sensation du joueur.

Dans le cadre d'un programme médical ou de bien-être, un objectif serait de contrôler son SNA afin de modifier la représentation et/ou le comportement et/ou les facultés de l'avatar de manière à atteindre un objectif. Par exemple, pour les enfants, un contrôle de la respiration impacterait en temps réel la représentation d'un avatar tel qu'un avion en vol, de sorte à stabiliser l'avion sur un écran, incitant de ce fait l'enfant à mieux maîtriser sa respiration. L'invention propose ainsi un système d'aide à la régulation du SNA.

Ainsi, la représentation visuelle en temps réel de l'activité du SNA d'un sujet permet de rétroagir pour modifier son SNA en temps réel. En fonction des applications, cette rétroaction peut être : commandée par la personne elle-même, par exemple pour réduire son stress ; ou commandée par une autre personne ou un système externe afin de fournir au sujet, comme décrit précédemment, des stimuli de respiration pour modifier son état émotionnel ou son niveau de stress.

En alternative à une représentation visuelle, l'invention peut également fournir une représentation sonore ou tactile des paramètres calculés et relatifs au SNA.

L'invention peut également cumuler une représentation visuelle ou sonore ou tactile des paramètres calculés et relatifs au SNA.

L'invention n'est pas limitée aux seuls modes et exemples de réalisation décrits ci-dessus, mais s'étend à tous les modes de réalisation entrant dans la portée des revendications.

## Revendications

**1.** Procédé de surveillance du système nerveux autonome (SNA) d'un sujet (10), le procédé comprenant les étapes suivantes :

• l'acquisition d'au moins un signal physiologique, ce signal physiologique comprenant W battements cardiaques, chaque battement cardiaque étant détecté à un instant t, avec t compris entre $t_{X-W+1}$ et $t_X$, l'instant $t_X$ étant le dernier battement des W battements cardiaques ;

• la génération d'une donnée qui est fonction de la variabilité de la fréquence cardiaque (VFC) sur l'ensemble des W battements cardiaques, la VFC comprenant W-1 intervalles RR séparant deux battements cardiaques consécutifs détectés respectivement aux instants $t_{k-1}$ et $t_k$, chaque intervalle RR présentant une durée d'une valeur $a_k = t_k - t_{k-1}$ avec k = (X-W+2)... X ; **caractérisé en ce qu'**il comprend :

• le calcul à l'aide d'au moins un microprocesseur des paramètres suivants:

- le paramètre TAS($t_X$) représentant le niveau d'activité du système sympathique et qui est fonction du rapport entre d'une part une première somme obtenue en sommant, parmi les valeurs $a_k$, uniquement celles qui sont supérieures à la valeur $a_{k-1}$ de l'intervalle immédiatement précédent et une deuxième somme obtenue en sommant toutes les W-1 valeurs $a_k$ ; et

- le paramètre TAP($t_X$) représentant le niveau d'activité du système parasympathique et qui est fonction du rapport entre d'une part une première somme obtenue en sommant, parmi les valeurs $a_k$, uniquement celles qui sont inférieures à la valeur $a_{k-1}$ de l'intervalle immédiatement précédent et une deuxième somme obtenue en sommant toutes les W-1 valeurs $a_k$ ; et

- le paramètre NS($t_X$) représentant un niveau de stress du sujet (10) à l'instant $t_X$ et calculé en appliquant l'équation suivante :

$$NS(t_X) = 100 + TAS(t_X) - TAP(t_X) \; ;$$

• la fourniture d'une donnée représentative dudit au moins un paramètre.

2. Procédé de surveillance selon la revendication précédente comprenant :

- la génération d'un stimulus de respiration fourni au sujet (10) pendant une durée T, le stimulus de respiration comprenant une consigne respiratoire pour que le sujet (10) respire de manière symétrique ;

- la durée T étant calculée en fonction d'au moins une donnée fonction du rythme cardiaque (RC) du sujet (10).

3. Procédé de surveillance selon la revendication précédente dans lequel l'au moins un paramètre comprend une valeur de stress chronique SC qui est fonction du paramètre NS($t_X$) pendant ladite durée T.

4. Procédé de surveillance selon la revendication précédente dans lequel la valeur de stress chronique SC est égale à la valeur moyenne du paramètre NS($t_X$) sur ladite durée T du stimulus de respiration.

5. Procédé de surveillance selon l'une quelconque des revendications précédentes dans lequel ledit au moins un paramètre comprenant un paramètre NSR($t_X$) relatif à un niveau de stress résiduel du sujet (10) à l'instant $t_X$ et calculé en appliquant l'équation suivante :

$$NSR(t_X) = NS(t_X) \cdot \frac{RC_{Repos}}{RC(t_X)}$$

dans laquelle $RC_{Repos}$ est la fréquence cardiaque au repos, c'est-à-dire le sujet (10) est inactif pendant une durée de repos comprise d'au moins 20 secondes et de préférence 40 secondes, RC($t_X$) est la fréquence cardiaque à l'instant $t_X$.

6. Procédé de surveillance selon l'une quelconque des revendications précédentes :

dans lequel ledit au moins un paramètre est le TAP($t_X$) représentant le niveau d'activité du système parasympathique et dans lequel le TAP($t_X$) est calculé en appliquant l'équation suivante :

$$TAP(t_X) = 100 \cdot \frac{\sum_{k=X-W+2}^{X} (t_k - t_{k-1}) \; si \; (a_k \geq a_{k-1})}{\sum_{k=X-W+2}^{X} (t_k - t_{k-1})}$$

ou dans lequel ledit au moins un paramètre est le $TPSP(t_X)$ et dans lequel le $TPSP(t_X)$ est calculé en appliquant l'équation suivante :

$$TPSP(t_X) = 100 \cdot \frac{\sum_{k=X-W+2}^{X} (t_k - t_{k-1}) \ si \ (a_k < a_{k-1} \ et \ a_{k-1} > a_{k-2} \ et \ a_{k+1} > a_k)}{\sum_{k=X-W+2}^{X} (t_k - t_{k-1})}$$

ou dans lequel ledit au moins un paramètre est le $TPPS(t_X)$ et dans lequel le $TPPS(t_X)$ est calculé en appliquant l'équation suivante :

$$TPPS(t_X) = 100 \cdot \frac{\sum_{k=X-W+2}^{X} (t_k - t_{k-1}) \ si \ (a_k > a_{k-1} \ et \ a_{k-1} < a_{k-2} \ et \ a_{k+1} < a_k)}{\sum_{k=X-W+2}^{X} (t_k - t_{k-1})}$$

Avec, pour chacune de ces équations:

- $a_{k-1}$ = valeur de l'intervalle de temps immédiatement précédent l'intervalle de temps de durée $a_k$, soit $a_{k-1} = t_{k-1} - t_{k-2}$.
- k = (X-W+2), (X-W+3)... X.

7. Procédé de surveillance selon l'une quelconque des revendications dans lequel l'étape d'acquisition d'au moins un signal physiologique est réalisée par au moins un des dispositifs suivants qui sont configurés pour être portés par le sujet (10) : un capteur d'électrocardiogramme (ECG), une ceinture thoracique, un bracelet ou une montre équipés d'un capteur photopléthysmographique.

8. Procédé de surveillance selon l'une quelconque des revendications précédentes dans lequel on calcule le paramètre $\overline{TPSP}$ représentant le taux approximé de pollution du système sympathique vers le système parasympathique et calculé en appliquant l'équation suivante :

$$\overline{TPSP} = 100 \cdot \frac{\sum_{n=1}^{N-1} 1 \ si \ (a_n < a_{n-1} \ et \ a_{n-1} > a_{n-2} \ et \ a_{n+1} > a_n)}{N-1}$$

ou dans lequel on calcule le paramètre $\overline{TPPS}$ représentant le taux approximé de pollution du système parasympathique vers le système sympathique et calculé en appliquant l'équation suivante :

$$\overline{TPPS} = 100 \cdot \frac{\sum_{n=1}^{N-1} 1 \ si \ (a_n > a_{n-1} \ et \ a_{n-1} < a_{n-2} \ et \ a_{n+1} < a_n)}{N-1}$$

9. Procédé de surveillance selon l'une quelconque des revendications précédentes dans lequel la fourniture d'une donnée représentative dudit au moins un paramètre comprend la fourniture d'une représentation visuelle et/ou auditive dudit au moins un paramètre ou dans lequel la fourniture d'une donnée représentative dudit au moins un paramètre comprend un affichage sur écran de l'un parmi les dispositifs suivants : une montre, un téléphone, un ordinateur portable, une tablette.

10. Procédé de surveillance selon l'une quelconque des revendications précédentes dans lequel ledit au moins paramètre est calculé en temps réel ou à intervalles réguliers et comprenant une étape de fourniture d'une représentation visuelle de l'évolution au cours du temps dudit au moins un paramètre.

11. Produit programme d'ordinateur comprenant des instructions, qui lorsqu'elles sont effectuées par au moins un processeur, exécute le procédé selon l'une quelconque des revendications précédentes.

12. Système de surveillance (100) du système nerveux autonome (SNA) d'un sujet (10), comprenant un capteur (110) configuré pour recevoir des données d'un signal physiologique comprenant W battements cardiaques, chaque battement cardiaque étant détecté à un instant t, avec t compris entre $t_{X-W+1}$ et $t_X$, l'instant $t_X$ étant le dernier battement des W battements cardiaques;
et comprenant au moins un module de traitement des données (222) équipé d'au moins un processeur configuré pour exécuter les étapes suivantes :

• génération d'une donnée qui est fonction de la variabilité de la fréquence cardiaque (VFC) sur l'ensemble des W battements cardiaques, la VFC comprenant W-1 intervalles RR séparant deux battements cardiaques consécutifs détectés respectivement aux instants $t_{k-1}$ et $t_k$, chaque intervalle RR présentant une durée d'une valeur $a_k = t_k - t_{k-1}$ avec k = (X-W+2)... X ; **caractérisé en ce qu'**il comprend :
• calcul des paramètres suivants:

- le paramètre TAS($t_X$) représentant le niveau d'activité du système sympathique et qui est fonction du rapport entre d'une part une première somme obtenue en sommant, parmi les valeurs $a_k$, uniquement celles qui sont supérieures à la valeur $a_{k-1}$ de l'intervalle immédiatement précédent et une deuxième somme obtenue en sommant toutes les W-1 valeurs $a_k$ ; et
- le paramètre TAP($t_X$) représentant le niveau d'activité du système parasympathique et qui est fonction du rapport entre d'une part une première somme obtenue en sommant, parmi les valeurs $a_k$, uniquement celles qui sont inférieures à la valeur $a_{k-1}$ de l'intervalle immédiatement précédent et une deuxième somme obtenue en sommant toutes les W-1 valeurs $a_k$ ; et
- le paramètre NS($t_X$) représentant un niveau de stress du sujet (10) à l'instant $t_X$ et calculé en appliquant l'équation suivante :

$$NS(t_X) = 100 + TAS(t_X) - TAP(t_X) \; ;$$

et comprenant un dispositif d'affichage (130), couplé au module de traitement des données (222) et configuré pour fournir au sujet (10) une donnée représentative dudit au moins un paramètre.

13. Système (100) de surveillance selon la revendication précédente dans lequel le capteur (110) est l'un des dispositifs suivants qui sont configurés pour être portés par le sujet (10) : un capteur d'électrocardiogramme (ECG), une ceinture thoracique, un dispositif photopléthysmographique installé dans un bracelet ou dans une montre.

14. Système (100) selon l'une quelconque des deux revendications précédentes dans lequel le module de traitement des données (222) est mécaniquement solidaire du capteur (110) ou dans lequel le module de traitement des données (222) est localisé à distance du capteur (110) et couplé au capteur (110) en utilisant un module de communication filaire ou sans fil.

15. Système (100) selon l'une quelconque des trois revendications précédentes dans lequel le dispositif d'affichage (130) est configuré pour réaliser une représentation visuelle dudit au moins un paramètre affichée sur un écran de l'un parmi les dispositifs suivants : une montre, un téléphone, un ordinateur portable, une tablette.

**Patentansprüche**

1. Verfahren zur Überwachung des autonomen Nervensystems (SNA) einer Person (10), wobei das Verfahren die folgenden Schritte umfasst:

• Erfassen mindestens eines physiologischen Signals, dieses physiologische Signal enthält W Herzschläge, jeder Herzschlag wird zu einem Zeitpunkt t erfasst, wobei t zwischen $t_{X-W+1}$ und $t_x$ liegt, und der Augenblick $t_x$ der letzte Herzschlag der W-Herzschläge ist,
• Erzeugen einer Angabe, die eine Funktion der Variabilität der Herzfrequenz (VFC) über alle W-Herzschläge hinweg ist, wobei die VFC W -1 RR-Intervalle aufweist, die zwei aufeinanderfolgende Herzschläge trennen, die jeweils zu den Zeitpunkten $t_{k-1}$ und $t_k$ erfasst wurden, wobei jedes RR-Intervall eine Dauer mit einem Wert von $a_k = t_k - t_{k-1}$ mit k = (X-W+2)... X aufweist; **dadurch gekennzeichnet, dass** es umfasst:
• Berechnung mittels mindestens eines Mikroprozessors der folgenden Parameter:

- der Parameter TAS ($t_x$), der das Aktivitätsniveau des sympathischen Systems darstellt, und der eine Funktion des Verhältnisses zwischen einerseits einer ersten Summe, die sich ergibt, wenn von den $a_k$-Werten nur die addiert werden, die höher sind als der Wert $a_{k-1}$ des unmittelbar vorangehenden Intervalls und eine zweite Summe, die sich ergibt durch Addition aller W-1 $a_k$-Werte; und
- der Parameter TAP ($t_x$), der das Aktivitätsniveau des parasympathischen Systems darstellt; und der eine Funktion des Verhältnisses zwischen einer ersten Summe, die sich ergibt, wenn von den $a_k$-Werten nur die addiert werden, die niedriger sind als der Wert $a_{k-1}$ des unmittelbar vorangehenden Intervalls und eine zweite Summe, die sich ergibt durch Addition aller W-1 $a_k$-Werte; und
- der Parameter NS($t_x$), der das Stressniveau der Person (10) zum Zeitpunkt $t_x$ darstellt und unter Verwendung der folgenden Gleichung berechnet wird:

$$\mathrm{NS}(t_X) = 100 + TAS(t_X) - TAP(t_X)$$

Bereitstellen einer Angabe, die für mindestens einen Parameter repräsentativ ist.

2. Überwachungsverfahren nach dem vorangehenden Anspruch, das umfasst:

- Erzeugung eines Atemstimulus, der der Person (10) während eines Zeitraums T, geliefert wird, wobei der Atemstimulus eine Atemvorgabe enthält, damit die Person (10) symmetrisch atmet;
- die Dauer T wird dabei in Abhängigkeit von mindestens einer Funktionsangabe des Herzrhythmus (RC) der Person (10) berechnet.

3. Überwachungsverfahren nach dem vorangehenden Anspruch, bei dem der mindestens eine Parameter einen chronischen Stresswert SC enthält, der eine Funktion des Parameters NS($t_x$) während dieses Zeitraums T ist.

4. Überwachungsverfahren nach dem vorangehenden Anspruch, bei dem der chronische Stresswert SC gleich dem Durchschnittswert des Parameters NS($t_x$) über diesen Zeitraum T des Atmungsstimulus hinweg, ist.

5. Überwachungsverfahren nach irgendeinem der vorangehenden Ansprüche, bei denen dieser mindestens eine Parameter einen Parameter NSR($t_x$) enthält, bezüglich eines verbleibenden Stressniveaus der Person (10) im Augenblick $t_x$ und berechnet, durch Anwenden der folgenden Gleichung:

$$\mathrm{NSR}(t_X) = \mathrm{NS}(t_X) \cdot \frac{RC_{\mathrm{Repos}}}{RC(t_X)}$$

Bei der $RC_{\mathrm{Repos}}$ die Herzfrequenz in Ruhe ist, das heißt die Person (10) ist während einer Ruhezeit von mindestens 20 Sekunden und vorzugsweise 40 Sekunden inaktiv, RC ($t_x$) ist die Herzfrequenz im Augenblick $t_x$.

6. Überwachungsverfahren nach irgendeinem der vorangehenden Ansprüche: darin steht TAP($t_x$) für das Aktivitätsniveau des parasympathischen Systems und bei dem der TAP($t_x$) berechnet wird durch Anwenden der folgenden Gleichung:

$$\mathrm{TAP}(t_X) = 100 \cdot \frac{\sum_{k=X-W+2}^{X}(t_k - t_{k-1})\ \mathrm{si}\ (a_k \geq a_{k-1})}{\sum_{k=X-W+2}^{X}(t_k - t_{k-1})}$$

oder in der dieser mindestens eine Parameter der TPSP($t_x$) ist, und in der der TPSP($t_x$) berechnet wird durch Anwenden der folgenden Gleichung:

$$\text{TPSP}(t_X) = 100 \cdot \frac{\sum_{k=X-W+2}^{X} (t_k - t_{k-1}) \; si \; (a_k < a_{k-1} \; et \; a_{k-1} > a_{k-2} \; et \; a_{k+1} > a_k)}{\sum_{k=X-W+2}^{X} (t_k - t_{k-1})}$$

oder in der dieser mindestens eine Parameter der TPPS($t_X$) ist, und in der der TPPS($t_X$) berechnet wird durch Anwenden der folgenden Gleichung:

$$\text{TPPS}(t_X) = 100 \cdot \frac{\sum_{k=X-W+2}^{X} (t_k - t_{k-1}) \; si \; (a_k > a_{k-1} \; et \; a_{k-1} < a_{k-2} \; et \; a_{k+1} < a_k)}{\sum_{k=X-W+2}^{X} (t_k - t_{k-1})}$$

Wobei, in jeder dieser Gleichungen:

- $a_{k-1}$ = Wert des unmittelbar vorangehenden Zeitintervalls mit der Dauer $a_k$, also $a_{k-1} = t_{k-1} - t_{k-2}$.
- $k$ = (X-W+2), (X-W+3)... X.

7. Überwachungsverfahren nach irgendeinem der vorangehenden Ansprüche, bei dem der Schritt des Erfassens mindestens eines physiologischen Signals, durch mindestens eines der folgenden Geräte durchgeführt wird, die dazu konfiguriert sind, von der Person (10) getragen zu werden: ein Elektrokardiogramm- (ECG)-Sensor, ein Brustgurt, ein Armband oder eine Uhr mit einem Pulswellensensor.

8. Überwachungsverfahren nach irgendeinem der vorangehenden Ansprüche, bei dem der Parameter $\overline{\text{TPSP}}$, der für die genäherte Belastungsrate des sympathischen Systems zum parasympathischen System steht, berechnet wird, durch Anwenden der folgenden Gleichung.

$$\overline{\text{TPSP}} = 100 \cdot \frac{\sum_{n=1}^{N-1} 1 \; si \; (a_n < a_{n-1} \; et \; a_{n-1} > a_{n-2} \; et \; a_{n+1} > a_n)}{N-1}$$

der Parameter $\overline{\text{TPPS}}$, der für die genäherte Belastungsrate des parasympathischen Systems zum sympathischen System steht, berechnet wird, durch Anwenden der folgenden Gleichung:

$$\overline{\text{TPPS}} = 100 \cdot \frac{\sum_{n=1}^{N-1} 1 \; si \; (a_n > a_{n-1} \; et \; a_{n-1} < a_{n-2} \; et \; a_{n+1} < a_n)}{N-1}$$

9. Überwachungsverfahren nach irgendeinem der vorangehenden Ansprüche, bei dem das Bereitstellen einer Angabe, die für diesen mindestens einen Parameter repräsentativ ist, das Bereitstellen einer visuellen und/ oder Audio-Darstellung dieses mindestens einen Parameters umfasst oder bei dem das Bereitstellen einer Angabe, die für diesen mindestens einen Parameter repräsentativ ist, die Anzeige auf dem Bildschirm mindestens eines der folgenden Geräte umfasst; eine Uhr, ein Telefon, ein Notebook, ein Tablet.

10. Überwachungsverfahren nach irgendeinem der vorangehenden Ansprüche, bei dem dieser mindestens eine Parameter in Realzeit oder mit regelmäßigen Intervallen berechnet wird und einen Schritt des Bereitstellens einer visuellen Darstellung über die Zeit hinweg dieses mindestens einen Parameters umfasst.

11. Computerprogramm mit Befehlen, die, wenn sie von mindestens einem Prozessor ausgeführt werden, das Verfahren nach irgendeinem der vorangehenden Ansprüche ausführen.

12. Überwachungssysteme (100) zur Überwachung des autonomen Nervensystems (SNA) einer Person (10), mit einem Sensor (110), konfiguriert zum Empfang der Daten eines physiologischen Signals, mit W-Herzschlägen, jeder Herzschlag wird zu einem Zeitpunkt t erfasst, wobei t zwischen $t_{X-W+1}$ und $t_X$ liegt, und der Augenblick $t_X$ der letzte Herzschlag der W- Herzschläge ist,

und mit mindestens einem Modul zur Datenverarbeitung (222), ausgerüstet mit mindestens einem Prozessor, konfiguriert zur Ausführung der folgenden Schritte:

• Erzeugen einer Angabe, die eine Funktion der Variabilität der Herzfrequenz VFC über alle W-Herzschläge hinweg ist, wobei die VFC W -1 RR-Intervalle aufweist, die zwei aufeinanderfolgende Herzschläge trennen, die jeweils zu den Zeitpunkten $t_{k-1}$ und $t_k$ erfasst wurden, wobei jedes RR-Intervall eine Dauer mit einem Wert von $a_k = t_k - t_{k-1}$ mit k = (X-W+2)... X aufweist; **dadurch gekennzeichnet, dass** es umfasst:
• Berechnung der folgenden Parameter:

- der Parameter TAS ($t_x$), der das Aktivitätsniveau des sympathischen Systems darstellt, und der eine Funktion ist des Verhältnisses zwischen einerseits einer ersten Summe, die sich ergibt, wenn von den $a_k$-Werten nur die addiert werden, die höher sind als der Wert $a_{k-1}$ des unmittelbar vorangehenden Intervalls und eine zweite Summe, die sich ergibt durch Addition aller W-1 $a_k$-Werte; und
- der Parameter TAP ($t_x$), der das Aktivitätsniveau des parasympathischen Systems darstellt; und der eine Funktion ist des Verhältnisses zwischen einer ersten Summe, die sich ergibt, wenn von den $a_k$-Werten nur die addiert werden, die niedriger sind als der Wert $a_{k-1}$ des unmittelbar vorangehenden Intervalls und eine zweite Summe, die sich ergibt durch Addition aller W-1 $a_k$-Werte; und
- der Parameter NS($t_x$), der das Stressniveau der Person (10) zum Zeitpunkt $t_x$ darstellt und unter Verwendung der folgenden Gleichung berechnet wird:

$$NS(t_X) = 100 + TAS(t_X) - TAP(t_X)$$

und umfassend eine Anzeigevorrichtung (130), verbunden mit dem Datenverarbeitungsmodul (222) und konfiguriert, um der Person (10) eine repräsentative Angabe dieses mindestens einen Parameters zu liefern.

13. Überwachungssystem (100) nach dem vorangehenden Anspruch, bei dem der Sensor (110) eines der folgenden Geräte ist, die dazu konfiguriert sind, von der Person (10) getragen zu werden: ein Elektrokardiogramm- (ECG)- Sensor, ein Brustgurt, ein Armband oder eine Uhr mit einem Pulswellensensor.

14. System (100) nach irgendeinem der beiden vorangehenden Ansprüche, bei denen das Datenverarbeitungsmodul (222) mechanisch mit dem Sensor (110) fest verbunden ist, oder bei dem sich das Datenverarbeitungsmodul (222) in einer Entfernung vom Sensor (110) befindet und mit dem Sensor (110) unter Verwendung eines drahtgebundenen oder drahtlosen Kommunikationsmoduls verbunden ist.

15. System (100) nach irgendeinem der drei vorangehenden Ansprüche, bei dem das Anzeigesystem (130) dazu konfiguriert ist, eine visuelle Darstellung dieses Parameters auf dem Bildschirm mindestens eines der folgenden Geräte anzuzeigen: eine Uhr, ein Telefon, ein Notebook, ein Tablet.

**Claims**

1. A method of monitoring the autonomic nervous system (SNA) of a subject (10), wherein the method comprises the following steps:

• the acquisition of at least one physiological signal, which comprises W heartbeats, each heartbeat being detected at an instant t, with t lying between $t_{x-w+1}$ and $t_x$, the instant $t_x$ being the last heartbeat of the W heartbeats;
• the generation of a data item which depends on the variability of the heart rate (VFC) with respect to all the W heartbeats, with VFC comprising W-1 RR intervals separating two consecutive heartbeats detected at the instants $t_{k-1}$ and $t_k$ respectively and each RR interval having a duration of $a_k = t_k - t_{k-1}$ where k = (X-W+2)... X; **characterized in that** it consists in:
• calculation of the following parameters using at least one microprocessor:

- the parameter TAS($t_x$), representing the level of activity of the sympathetic system and which depends on the ratio on the one hand between a first sum obtained by summing, among the $a_k$ values, only those which are greater than the value $a_{k-1}$ of the immediately preceding interval and a second sum obtained by summing all the W-1 values $a_k$; and on the other
- the parameter TAP($t_x$) representing the activity level of the parasympathetic system which depends on the ratio between, on the one hand, a first sum obtained by summing, among the $a_k$ values, only those which are less than the value $a_{k-1}$ of the immediately preceding interval and a second sum obtained by summing all the W-1 values $a_k$; and on the other hand
- the parameter $NS(t_x)$ representing a stress level of subject (10) at instant $t_x$ and calculated by applying the following equation:

$$NS(t_X) = 100 + TAS(t_X) - TAP(t_X)$$

providing data representative of the said at least one parameter.

2. A monitoring method according to the preceding claim comprising:

- the generation of a breathing stimulus supplied to the subject (10) for a time T, which breathing stimulus comprises a respiratory indication for the subject (10) to breathe symmetrically; the time T being calculated depending on at least one data item depending on the heart rate (RC) of the subject (10).

3. A monitoring method according to the preceding claim wherein the at least one parameter comprises a chronic stress value SC which depends on the parameter $NS(t_x)$ during the said duration T.

4. A monitoring method according to the preceding claim wherein the chronic stress value SC equals the mean value of the parameter $NS(t_x)$ over said duration T of the breathing stimulus.

5. A monitoring method according to any of the preceding claims wherein the said at least one parameter comprises a parameter $NSR(t_x)$ relating to a stress residue level of the subject (10) at the time $t_x$ and calculated by applying the following equation:

$$NSR(t_X) = NS(t_X) \cdot \frac{RC_{Repos}}{RC(t_X)}$$

In which $RC_{Repos}$ is the resting heart rate, i.e. when the subject (10) is inactive for a rest period of at least 20 seconds and preferably 40 seconds, wherein $CR(t_x)$ is the heart rate at instant $t_x$.

6. A monitoring method according to any of the preceding claims wherein the said at least one parameter comprises a parameter $TAP(t_x)$ relating to a parasympathetic system activity level of the subject at the time $TAP(t_x)$ calculated by applying the following equation:

$$TAP(t_X) = 100 \cdot \frac{\sum_{k=X-W+2}^{X} (t_k - t_{k-1}) \ si \ (a_k \geq a_{k-1})}{\sum_{k=X-W+2}^{X} (t_k - t_{k-1})} \quad if \ (ak \geq a_{k-1})$$

or wherein the said at least one parameter is $TPSP(t_x)$ and wherein the $TPST(t_x)$ is calculated by applying the following equation:

$$TPSP(t_X) = 100 \cdot \frac{\sum_{k=X-W+2}^{X} (t_k - t_{k-1}) \ si \ (a_k < a_{k-1} \ et \ a_{k-1} > a_{k-2} \ et \ a_{k+1} > a_k)}{\sum_{k=X-W+2}^{X} (t_k - t_{k-1})}$$

or wherein the said at least one parameter is $TPPS(t_x)$ and wherein the $TPPS(t_x)$ is calculated by applying the following equation:

$$TPPS(t_X) = 100 \cdot \frac{\sum_{k=X-W+2}^{X} (t_k - t_{k-1}) \ si \ (a_k > a_{k-1} \ et \ a_{k-1} < a_{k-2} \ et \ a_{k+1} < a_k)}{\sum_{k=X-W+2}^{X} (t_k - t_{k-1})}$$

With, for each of these equations:

- $a_{k-1}$ = value of the time interval immediately preceding the time interval having duration $a_k$, i.e. $a_{k-1} = t_{k-1} - t_{k-2}$
- k = (X-W+2), (X-W+3) ... X.

7. A monitoring method according to any one of the claims wherein the step of acquiring at least one physiological signal is performed by at least one of the following devices which are configured to be worn by the subject (10): an electrocardiogram (ECG) sensor, a chest belt, a bracelet or a watch equipped with a photoplethysmographic sensor.

8. A monitoring method according to any of the preceding claims wherein the parameter $\overline{TPSP}$ representing the approximate pollution rate from the sympathetic to the parasympathetic system is calculated by applying the following equation:

$$\overline{TPSP} = 100 \cdot \frac{\sum_{n=1}^{N-1} 1 \ si \ (a_n < a_{n-1} \ et \ a_{n-1} > a_{n-2} \ et \ a_{n+1} > a_n)}{N-1}$$

or wherein the $\overline{TPPS}$ parameter representing the approximate pollution rate from the parasympathetic to the sympathetic system is calculated by applying the following equation:

$$\overline{TPPS} = 100 \cdot \frac{\sum_{n=1}^{N-1} 1 \ si \ (a_n > a_{n-1} \ et \ a_{n-1} < a_{n-2} \ et \ a_{n+1} < a_n)}{N-1}$$

9. A method of monitoring according to any of the preceding claims wherein the provision of data representative of the said at least one parameter comprises the provision of a visual and/or aural representation of the said at least one parameter or wherein the provision of data representative of the said at least one parameter includes an on-screen display of one of the following devices: a watch, a telephone, a laptop, a tablet.

10. A method of monitoring according to any of the preceding claims wherein the said at least one parameter is calculated in real time or at regular intervals and comprising a step of providing a visual representation of the evolution of the said at least one parameter with time.

11. A computer program product comprising instructions, which when executed by at least one processor, executes the method according to any of the preceding claims.

12. A monitoring system (100) of the autonomic nervous system (SNA) of a subject (10), comprising a sensor (110) configured to receive data for a physiological signal comprising W heartbeats, each heartbeat being sensed at an instant t, with t ranging from $t_{x-w+1}$ to $t_x$, the instant $t_x$ being the last of the W heartbeats; and comprising at least one data processing module (222) with at least one processor configured to perform the following steps:

• the generation of a data item which depends on the variability of the heart rate (VFC) with respect to all the W heartbeats, with the VFC comprising W-1 RR intervals separating two consecutive heartbeats detected at the instants $t_{k-1}$ and $t_k$ respectively and each RR interval having a duration of $a_k = t_k - t_{k-1}$ where k = (X-W+2)... X; **characterized in that** it consists in:
• calculation of the following parameters:

- the parameter $TAS(t_x)$, representing the level of activity of the sympathetic system and which depends on the ratio on the one hand between a first sum obtained by summing, among the $a_k$ values, only those which are greater than the value $a_{k-1}$ of the immediately preceding interval and a second sum obtained by summing all the W-1 values $a_k$; and on the other

- the parameter TAP($t_x$) representing the activity level of the parasympathetic system which depends on the ratio between, on the one hand, a first sum obtained by summing, among the $a_k$ values, only those which are less than the value $a_{k-1}$ of the immediately preceding Interval and a second sum obtained by summing all the W-1 $a_k$ values; and
- the parameter NS($t_x$) representing a stress level of subject (10) at instant $t_x$ and calculated by applying the following equation:

$$NS(t_X) = 100 + TAS(t_X) - TAP(t_X)$$

and comprising a display device (130), coupled to the data processing module (222) and configured to provide the subject (10) with data representative of the at least one parameter.

13. A monitoring system (100) according to the preceding claim wherein the sensor (110) is one of the following devices which are configured to be worn by the subject (10): an electrocardiogram (ECG) sensor, a chest belt, a photoplethysmographic device installed in a wristband or in a watch.

14. A system (100) according to either of the two preceding claims wherein the data processing module (222) is mechanically integral with the sensor (110) or wherein the data processing module (222) is remotely located from the sensor (110) and couples to the sensor (110) using a wired or wireless communication module.

15. A system (100) according to any one of the three preceding claims wherein the display device (130) is configured to perform a visual representation of said at least one parameter displayed on a screen of one of the following devices: a watch, a telephone, a laptop, a tablet.

Application
visuelle,
auditive

130

Calcul de TAS,
TAP, TPSP,
TPPS, Stress
total, Stress
résiduel

222

10

113

110

100

**Figure 1**

**Figure 2(a)**

**Figure 2(b)**

**Figure 3**

Temps (secondes)

**Figure 4**

Spectre HRV

Spectre HRV

**Figure 5(a)**

**Figure 5(b)**

VFC sans faux battements

**Figure 6(a)**

VFC avec 2 faux battements

**Figure 6(b)**

**Figure 6(c)**

**Figure 6(d)**

**Figure 7(a)**

**Figure 7(b)**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 7079888 B
- US 20130009779 A1

- US 20130079652 A1